(19) Europäisches Patentamt
European Patent Office
Office européen des brevets

(11) **EP 2 648 123 A1**

(12) **EUROPEAN PATENT APPLICATION**

(43) Date of publication:
**09.10.2013 Bulletin 2013/41**

(51) Int Cl.:
**G06F 19/00** (2011.01)

(21) Application number: **13162518.8**

(22) Date of filing: **05.04.2013**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(30) Priority: **08.04.2012 KR 20120036465**<br><br>(71) Applicant: **Samsung Electronics Co., Ltd Gyeonggi-do 443-742 (KR)** | (72) Inventors:<br>• **Kim, Hyun-young**<br> **Gyeonggi-do (KR)**<br>• **Wi, Tae-hwan**<br> **Gyeonggi-do (KR)**<br><br>(74) Representative: **Birchenough, Lewis Harrison Goddard Foote LLP Saviour House 9 St Saviourgate York YO1 8NQ (GB)** |

(54) **User terminal device and system for performing user customized health management, and methods thereof**

(57)      A user health management method and device are provided. The user health management method includes receiving data from a data source using short-range wireless communication, the data indicating health-related information, determining health-related behavior information of a user based on the health-related information, generating a health care recommendation for the user based on the health-related behavior information, and outputting the health care recommendation.

FIG. 9

EP 2 648 123 A1

**Description**

BACKGROUND

**Field**

The present invention relates generally to apparatuses, terminal devices, systems, and methods for user customized health care. More particularly, although not exclusively, the present invention relates to a method, a terminal device, and a health management system for providing health care and health care recommendations using behavior guidelines of the user and behavior information of the user.

**Description of the Related Art**

Due to advances in medical technology, the human life span is increasing. To manage the health of people such as the elderly, the chronically ill, the obese, and patients with adult diseases, continuous health management is needed. For example, people with chronic conditions or illnesses such as high blood pressure or diabetes need constant health care to manage their condition.

However, people with these chronic conditions or illnesses generally treat their disease by taking drugs prescribed by a doctor over the long term, rather than receiving treatment in a hospital. In addition to medication, these diseases may be treated with exercise and diet. However, it is not always easy to manage the treatment of these conditions without additional help and information such as reminders and updated treatment plans.

There has been a great increase in people who desire to maintain a healthy lifestyle by exercising and dieting to keep in shape, to stay healthy, and to prevent diseases.

The aging population, the increase of the chronically ill, and the increasing interest in a healthy lifestyle has created a need for personal healthcare management devices that provide customized user health management.

**SUMMARY**

It is an aim of certain embodiments of the present invention to address, solve, mitigate or obviate, at least partly, at least one of the problems and/or disadvantages associated with the related art, for example one or more of the problems and/or disadvantages mentioned above. Certain embodiments aim to provide at least one of the advantages described below.

Accordingly, one or more exemplary embodiments provide a user terminal device, a health management system, and a method for user customized health management so that the user can effectively care for his/her health.

According to an aspect of the present invention, there is provided a user health management method comprising the steps of: receiving data from a data source (e.g. using wireless communication, for example short-range wireless communication), the data indicating health-related information; determining health-related behavior information of a user based on the health-related information; generating a health care recommendation for the user based on the health-related behavior information; and outputting the health care recommendation.

In an exemplary embodiment, the user health management method may also comprise the steps of: receiving profile information including at least one of a weight, an age, a height, and a medical history of the user; and generating and setting a behavior guideline based on the profile information. In an exemplary embodiment, the step of generating the health care recommendation for the user based on the health-related behavior information may further comprise the step of: evaluating the health-related behavior information based on the behavior guideline.

In an exemplary embodiment, the step of generating a health care recommendation for the user based on the health-related behavior information may further comprise the step of: determining an evaluation score based on the evaluating the health-related behavior information.

In an exemplary embodiment, the step of determining the evaluation score based on the evaluating the health-related behavior information may comprise the steps of: adding positive points or a first score to the evaluation score if the health-related behavior information satisfies the behavior guideline; and adding negative points or a second score lower than the first score to the evaluation score if the health-related behavior information does not satisfy the behavior guideline.

In an exemplary embodiment, the step of generating the health care recommendation for the user based on the health-related behavior information may further comprise the steps of: determining whether the evaluation score is greater than or equal to a predetermined threshold; and generating an adjusted behavior guideline based on a result of the step of determining whether the evaluation score is greater than or equal to the predetermined threshold.

In an exemplary embodiment, the step of generating the adjusted behavior guideline may comprise the steps of: increasing an intensity of the behavior guideline if the evaluation score is greater than or equal to the predetermined threshold; and decreasing the intensity of the behavior guideline if the evaluation score is not greater than or equal to the predetermined threshold.

In an exemplary embodiment, the profile information may be received from at least one of the user, another user, or an external device.

In an exemplary embodiment, the health care recommendation may comprise a new behavior guideline for the user.

In an exemplary embodiment, the new behavior guideline may be a temporary behavior guideline requiring approval.

In an exemplary embodiment, the user health management method may further comprise the steps of: transmitting the new behavior guideline to a second device of another user authorized to provide the approval; and receiving the approval corresponding to the transmitted new behavior guideline from the second device. In an exemplary embodiment, the step of outputting the health care recommendation may further comprise the step of: outputting the new behavior guideline after receiving the approval.

In an exemplary embodiment, the data source may be a tag attached to an object.

In an exemplary embodiment, the short-range wireless communication may comprise at least one of near field communication, reading a barcode, reading a Quick Response code, Wi-Fi communication, and Zigbee communication.

In an exemplary embodiment, the step of outputting the health care recommendation may further comprise the step of: outputting at least one of: a graph based on the health-related behavior information; a notification based on the health-related behavior information; and an evaluation score based on the health-related behavior information.

In an exemplary embodiment, the data source may be an external sensor which monitors at least one of weight, body temperature, heart rate, blood pressure, blood sugar, speed, acceleration, and movement of the user.

According to another aspect of the present invention, there is provided a non-transitory computer readable medium having recorded thereon computer executable instructions that, when executed by a computer, cause the computer to perform a user health management method in accordance with the above-described aspects and/or embodiments.

According to another aspect of the present invention, there is provided a user health management device comprising: a communication module (e.g. a wireless communication device, for example a short-range wireless communication device) for receiving data from a data source (e.g. using wireless communication, for example short-range wireless communication), the data indicating health-related information; and a controller for determining health-related behavior information of a user based on the health-related information, generating a health care recommendation for the user based on the health-related behavior information, and outputting the health care recommendation.

In an exemplary embodiment, the user health management device may also comprise: an input for receiving profile information including at least one of a weight, an age, a height, and a medical history of the user. In an exemplary embodiment, the controller may be configured for generating and setting a behavior guideline based on the profile information, and generating the health care recommendation by evaluating the health-related behavior information based on the behavior guideline.

In an exemplary embodiment, the controller may be configured for generating the health care recommendation by determining an evaluation score based on the evaluating.

In an exemplary embodiment, the controller may be configured for determining the evaluation score by adding one of positive points or a first score to the evaluation score if the health-related behavior information satisfies the behavior guideline, and adding one of negative points or a second score lower than the first score to the evaluation score if the health-related behavior information does not satisfy the behavior guideline.

In an exemplary embodiment, the controller may be configured for determining whether the evaluation score is greater than or equal to a predetermined threshold and generating an adjusted behavior guideline based on the determining whether the evaluation score is greater than or equal to the predetermined threshold.

In an exemplary embodiment, the controller may be configured for generating an adjusted behavior guideline that increases an intensity of the behavior guideline if the evaluation score is greater than or equal to the predetermined threshold, and generating an adjusted behavior guideline that decreases the intensity of the behavior guideline if the evaluation score is not greater than or equal to the predetermined threshold.

In an exemplary embodiment, the input may be configured for receiving the profile information from at least one of the user, a second user, or an external device.

In an exemplary embodiment, the health care recommendation may comprise a new behavior guideline for the user.

In an exemplary embodiment, the new behavior guideline may comprise a temporary behavior guideline requiring approval.

In an exemplary embodiment, the controller may be configured for transmitting the new behavior guideline to a second device of a second user authorized to provide the approval, receiving the approval corresponding to the transmitted new behavior guideline from the second device, and outputting the health care recommendation including the new behavior guideline in response to receiving the approval.

In an exemplary embodiment, the data source may be a tag attached to an object.

In an exemplary embodiment, the short-range wireless communication may be at least one of near field communication, reading a barcode, reading a Quick Response code, Wi-Fi communication, and Zigbee communication.

In an exemplary embodiment, the user health management device may also comprise a display for displaying the health care recommendation. The health care recommendation may comprise at least one of a graph, a notification, and an evaluation score.

In an exemplary embodiment, the communication module may be configured for receiving data from an external sensor for monitoring at least one of weight, body temperature, heart rate, blood pressure, blood sugar, speed, acceleration, g-force, and movement of the user.

In an exemplary embodiment, the communication module may be configured for receiving data from a plurality of external sensors forming a personal area network.

Another aspect of the present invention provides a computer program comprising instructions arranged, when executed, to implement a method, system and/or apparatus, in accordance with any one of the above-described aspects and/or embodiments. A further aspect provides machine-readable storage storing such a program.

Other aspects, advantages, and salient features of the invention will become apparent to those skilled in the art from the following detailed description, which, taken in conjunction with the annexed drawings, disclose exemplary embodiments of the invention.

## BRIEF DESCRIPTION OF THE DRAWING FIGURES

The above and other aspects, and features and advantages of certain exemplary embodiments and aspects of the present inventive will become apparent and more readily appreciated from the following description of exemplary embodiments, taken in conjunction with the accompanying drawings of which:

FIG. 1 is a diagram of a health management system according to an exemplary embodiment;

FIG. 2 is a block diagram of a user terminal device according to an exemplary embodiment;

FIG. 3 is a diagram of a method for collecting data of an external object in the user terminal device according to an exemplary embodiment;

FIG. 4 is a diagram of a method of the user terminal device for updating behavior information and health care information by tagging with the external object according to an exemplary embodiment;

FIG. 5 is a block diagram of a system server according to an exemplary embodiment;

FIGS. 6 through 12 are diagrams of operations of the health management system according to various exemplary embodiments;

FIG. 13 is a block diagram of a user terminal device according to another exemplary embodiment;

FIG. 14 is a diagram of an input screen for inputting user's basic information;

FIG. 15 is a diagram of a behavior guideline input screen displayed to input a behavior guideline;

FIG. 16 is a diagram of a screen displayed to modify the user's behavior guideline;

FIG. 17 is a diagram of a screen for checking user's health care information;

FIGS. 18 through 29 are diagrams of various screens displayed in the user terminal device;

FIG. 30 is a flowchart of a user customized health management method of the user terminal device according to an exemplary embodiment;

FIG. 31 is a flowchart of a method for setting the behavior guideline;

FIG. 32 is a flowchart of a method for receiving from a second device and storing or updating the behavior guideline;

FIG. 33 is a flowchart of a user customized health management method of the system server according to an exemplary embodiment;

FIG. 34 is a diagram of a health management system according to another exemplary embodiment;

FIG. 35 is a diagram of a method for generating behavior information in the user terminal device according to another exemplary embodiment;

FIG. 36 is a diagram of a method for informing of a user's status using health care information in the user terminal device according to another exemplary embodiment;

FIG. 37 is a diagram of a method for collecting data from an information collector using the user terminal device which is implemented using a mobile phone;

FIG. 38 is a block diagram of the information collector which provides sensing information to the user terminal device;

FIG. 39 is a block diagram of a user terminal device including a sensor and a micro-server therein;

FIG. 40 is a diagram of a system including the system server which is implemented using a cloud server;

FIG. 41 is a block diagram of a first device according to an exemplary embodiment;

FIG. 42 is a detailed block diagram of a controller; and

FIG. 43 is a diagram of software architecture stored in the first device.

## DETAILED DESCRIPTION OF EXEMPLARY EMBODIMENTS

The following description of exemplary embodiments of the present invention, with reference to the accompanying drawings, is provided to assist in a comprehensive understanding of the present invention, as defined by the claims. The description includes various specific details to assist in that understanding but these are to be regarded as merely exemplary. Accordingly, those of ordinary skill in the art will recognize that various changes and modifications of the embodiments described herein can be made without departing from the scope of the invention.

The terms and words used in the following description and claims are not limited to the bibliographical meanings, but, are merely used by the inventor to enable a clear and consistent understanding of the invention. Accordingly, it should be apparent to those skilled in the art that the following description of exemplary embodiments of the present invention is provided for illustration purpose only and not for the purpose of limiting the invention as defined by the appended claims.

The same or similar components may be designated by the same or similar reference numerals although they may be illustrated in different drawings. Detailed descriptions of structures, constructions, functions or processes known in the art may be omitted for clarity and conciseness, and to avoid obscuring the subject matter of the present invention.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, it is to be understood that the singular forms "a," "an," and "the" include plural referents unless the context clearly dictates otherwise. Thus, for example, reference to "an object" includes reference to one or more of such objects.

Features, integers, characteristics, compounds, chemical moieties or groups described in conjunction with a particular aspect, embodiment or example of the invention are to be understood to be applicable to any other aspect, embodiment or example described herein unless incompatible therewith.

It will be also be appreciated that, throughout the description and claims of this specification, language in the general form of "X for Y" (where Y is some action, activity or step and X is some means for carrying out that action, activity or step) encompasses means X adapted or arranged specifically, but not exclusively, to do Y.

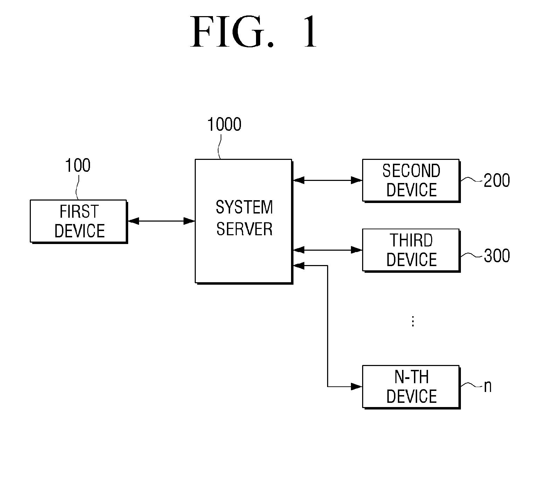

FIG. 1 is a diagram of a health management system according to an exemplary embodiment. Referring to FIG. 1, the health management system includes a first device 100, a system server 1000, a second device 200, and a third device 300 through an n-th device n.

The first device 100 can generate behavior information by collecting data relating to a behavior of a user. The behavior information can be generated for various items such as drug administration, exercise, and living environment etc. The behavior information can be processed in real time, or generated at regular intervals.

The system server 1000 provides a user customized health management service to a user of the first device 100 according to various information collected and transmitted by the first device 100. In this case, the system server 1000 can provide the user customized health management service in association with the second device 200, the third device 300, and the n-th device n.

In detail, the system of FIG. 1 establishes the behavior guideline (e.g., a health care recommendation) appropriate to the user, determines whether the user's actual behavior meets the behavior guideline, and generates health care information (e.g., a new health care recommendation) based on the determination. The health care information may be a new behavior guideline modified from a previous behavior guideline according to the user's behavior, and feedback information including an evaluation score by determining whether the behavior guideline and the behavior information match. The evaluation score can be determined based on various types of information that are scored and used to assign points such as life habit points and body part points, and that are accumulated and totaled. The life habit points score various habits in the user's daily life, such as a user's eating habits, exercise habits, sleep habits, drinking habits, and smoking habits, etc. For example, life habit points can be added or subtracted based on the calorie count of a meal, the number of meals, and irregular meal times. Additionally, low scores or high scores may be assigned according to the habits of users. When the user eats the normal meal amount at a fixed meal time, positive point can be added and a high score corresponding to eating habits may be assigned. For unhealthy habits such as drinking alcohol or smoking, low scores may be assigned to the user or negative points may be given to a user. When a smoker quits smoking, positive points may be added in proportion to a period of time in which the user does not smoke. The body part points are scores assigned to a good behavior and a bad behavior corresponding to a body part of the user. The body part may be classified as external body parts such as skin, lower body, abdomen, and head of the user, and as various internal organs such as heart, lung and brain. For example, when the smoking behavior is detected, negative points can be added to the point total of the stomach or the liver, or a low score may be assigned to the point total of the stomach and liver. When the smoking is continued, a low score can be assigned to the organ such as lungs or a cumulative score can be subtracted. When the same drug is taken for a long time causing harm a particular organ, a low score can be given to the corresponding organ. In this case, a supervisor such as doctor can check the body part score and take an appropriate action such as modifying the prescription.

In a case in which there is a prescription relating to the user's behavior information, the user's behavior is scored by taking into consideration the health effects of the prescription on the other items corresponding of the user's behavior information. In contrast, when there is no prescription relating to the user's behavior information, the user's behavior is scored based on behavior guidelines defining exercise, food, medication, and environmental standards corresponding to the user without taking into consideration the effects of the prescription. Accordingly, various feedback may be provided based on the health care information so as to allow users to continuously manage their health.

The behavior guidelines may include at least one of a first behavior guideline effective with an external expert's approval, and a second behavior guideline effective without the approval. For example, the first behavior guideline can include a prescription, and the second behavior guideline can include at least one of a food intake manner, a diet, an exercise method, an exercise volume, an exercise time, and a sleeping time.

The behavior guideline can include a long-term behavior guideline and a short-term behavior guideline. Herein, the short-term behavior guideline can be a shorter term behavior guideline which is specified as part of the long-term behavior guideline. For example, the short-term behavior guideline can be defined in detail with respect to one or more of a medication, eating habits, exercise habits, and life habits. The long-term behavior guideline may be a goal that is set to be reached over a longer period of time than the period of time set for short term behavior guidelines. A long-term behavior guideline may correspond to the weight of the user, a fitness level, a cure for a disease, and maintaining an exercise pattern or goal.

For example, when the long-term behavior guideline corresponds to exercise, exercising regularly over long period of time increases a long-term goal value. However, when the user neglects to exercise for a long period of time, this may decrease long term goal value and other guidelines to replace exercise may be suggested.

When the long-term behavior guideline is directed to curing a disease, the short-term behavior guideline may define what steps to take and how to perform the steps over a certain period of time in order to treat the disease. When the long-term behavior guideline is directed to fitness such as a weight loss goal, the short-term behavior guideline can determine short term weight-loss goals within the long-term weight loss by stages and define a calorie intake and an exercise volume to achieve the goal per stage.

When the user's behavior information is not input, the long-term behavior guideline can be established based on user's health information or other basic information of the user which is input by the user or a supervisor. Alternatively, when the behavior information is input, the long-term behavior guideline can be established according to a predefined algorithm based on the cumulative point totals of the behavior information. The user's basic information, behavior information, or cumulative point total can be transmitted to an external device so that the external device can establish and send the behavior guideline. For example, the long-term behavior guideline can include a prescription for the long-term treatment of a disease, an exercise plan, and a daily schedule chart, and the short-term behavior guideline can include a prescription for the short-term administration and a short-term diet plan chart.

Such behavior guidelines may be diverse, and the health management service appropriate to the user can be provided according to the behavior guideline. The behavior guidelines can be prioritized according to their importance. For example, the administration of a prescription can be given a first priority, the exercise method can be given a second priority, and the food intake manner can be given a third priority. The behavior guidelines may be generated sequentially according to their priority.

To provide the user customized health management service, the first device 100, the system server 1000, the second device 200, and the third device 300 of the system of FIG. 1 can work together. Detailed operations of the devices can be implemented differently according to various exemplary embodiments, to be explained. Alternatively, in addition to the system of FIG. 1, a single device may manage the user's health by itself.

To collect the data about the user's behavior, the first device 100 can be implemented using a terminal device of the user. In detail, the first device 100 can be implemented using a variety of portable devices such as mobile phone, personal digital assistant (PDA), tablet, personal computer (PC), MP3 player, and a calorie tracker. The first device 100 may be implemented using a wearable watch or a bracelet of the user. The first device 100 can collect the data in various manners.

For example, the first device 100 can collect the data using a communication method, for example a short-range wireless communication method. For example, in certain embodiments, short-range communication may be regarded as communications up to a certain separation (e.g. 1cm, 10cm, 1m, 10m, etc., or any other suitable range, according to the implementation). In the case of short-range wireless communication, the first device 100 can include a short-range wireless communication reader (e.g., short-range wireless communication device). Accordingly, the first device 100 can read the data by accessing an external object attached with a short-range wireless communication tag that is near the device. The short-range access works by moving at least one of the short-range wireless communication tag and reader to a location near the other causing the reader and the tag to within a communication range. This may be referred to as tagging. While in the communication range, the short-range wireless communication reader can read information recorded in the short-range wireless communication tag.

The short-range wireless communication method can include Near Field Communication (NFC). The NFC is a contactless short-range wireless communication technology using a frequency band of 13.56 MHz. Using NFC, data can be transmitted and received when one or more terminals approach a tag or each other, for example, within 10 cm or so. The short-range wireless communication method may also use, for example, barcode readers, Quick Response (QR) code readers, Wi-Fi, Zigbee, and Bluetooth, etc.

The collected data can relate to various user behaviors such as medication, food intake, exercise, and sleep. The data collection according to the short-range wireless communication method and the prediction of the user behavior using the collected data will be described in detail.

The user may directly input the data into the first device 100. For example, when the first device 100 includes an input means such as touch screen, touch pad, and/or button or is connected to an input means such as remote control, keyboard, and/or mouse, the user can directly input the data using such an input means. The input data reflects the user's actual behavior.

The user may connect an external storage medium such as a Universal Serial Bus (USB) memory device or a memory card to the first device 100 and send data stored in the external storage medium to the first device 100.

Alternatively, the user may connect other terminal devices such as a PC, laptop computer, tablet, and mobile phone to the first device 100 and send data to the first device 100 using the connected terminal device. As such, the first device 100 can collect various data relating to the user's behavior from various input devices.

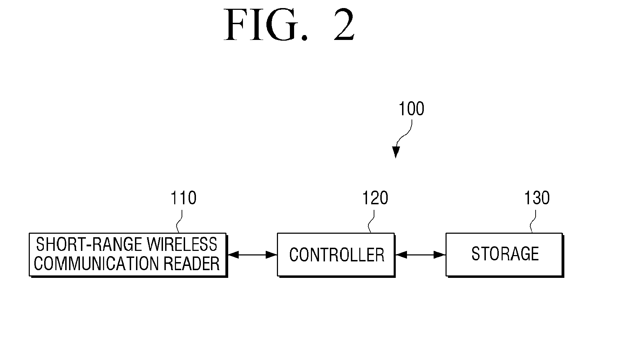

FIG. 2 is a block diagram of the first device 100 using the short-range wireless communication scheme according to an exemplary embodiment. In detail, the first device 100 is implemented using a user terminal device. Referring to FIG. 2, the first device 100 includes a short-range wireless communication reader 110, a controller 120, and storage 130.

When accessing external objects including a short-range wireless communication tag in the short range, the short-range wireless communication reader 110 reads and provides information recorded in the tag to the controller 120. The short-range wireless communication reader 110 can include a Radio Frequency (RF) module and an antenna coil. The short-range wireless communication reader 110 emits electromagnetic waves through the antenna coil. Hence, currents are induced by electromagnetic inductions in the short-range wireless communication tag (not shown) attached to the external object within an electromagnetic wave propagation distance based on the electromagnetic waves emitted from the first device 100. Thus, an integrated circuit in the short-range wireless communication tag is driven to send an RF signal including the stored data. The RF module in the short-range wireless communication reader 110 receives the RF signal through the antenna coil, demodulates and decodes the received RF signal, and detects the data in the RF signal. While only the short-range wireless communication reader 110 is depicted in FIG. 2, the first device 100 may include, if necessary, a short-range wireless communication module including the short-range wireless communication tag.

The controller 120 generates the user behavior information using the detected data. In this case, the behavior information can be generated by estimating the user's behavior according to the collected data. For example, when the short-range wireless communication tag attached to a medicine bottle is accessed by the terminal device through short-range communication, drug information in the tag is detected. Upon detecting the drug information, the controller 120 can recognize the administration of the corresponding drug and store the behavior information together with the administration time and the drug name. When the short-range wireless communication tag attached to a food container is accessed by the terminal device through short-range communication, the controller 120 can recognize the intake of the corresponding food. When the food container is a rice bowl, the controller 120 can recognize the intake of the meal corresponding to a size of the rice bowl, the calorie count of the rice bowl, and store the behavior information together with the meal time, the food type, and the intake quantity. The detected behavior information may be modified by a user according to the user's actual intake.

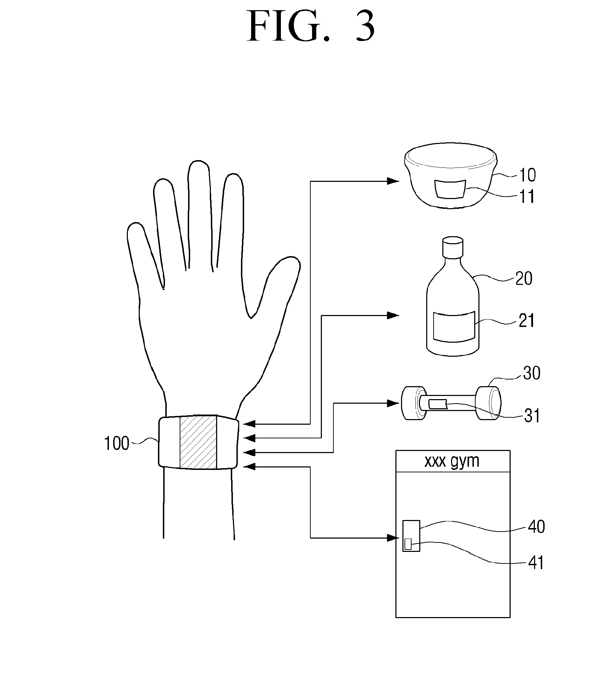

FIG. 3 is a diagram of a method for collecting data to generate the behavior information. Referring to FIG. 3, the user can wear the first device 100 on his/her wrist. The user brings the first device 100 close to external objects 10 through 40 attached with short-range wireless communication tags 11 through 41, and the first device 100 receives data recorded in the tags 11 through 41. For example, when the first object 10 being a food container approaches, the controller 120 generates food intake information using the data received from the food container tag 11. When the second object 20 being a medicine bottle approaches, the controller 120 generates medication information using data received from the medicine bottle tag 21. When the third object 30 being exercise equipment or the fourth object 40 being a door or lock of the gym approaches, the controller 120 generates exercise information using the data from exercise equipment tag 31 and gym door tag 41. The behavior information includes the generated food intake information, medication information, exercise information.

In addition to the data obtained using the short-range wireless communication, information added by the user can be included in the generation of the behavior information. For example, when the device of the user communicates with a tag of an object by mistake and the data is received, the controller 120 can output a message asking whether the user actually has conducted the corresponding behavior. The user may confirm, reject, or modify the behavior corresponding to the message and the controller 120 may recognize the actual behavior of the user based upon the user's input and generate the behavior information.

The controller 120 stores the generated behavior information in the storage 130. The controller 120 can store the behavior information by processing fields of the collected data into the storage format of the first device 100. For example, when the size of the behavior information is greater than a bit size of a region allocated to record the behavior information in the storage 130, the controller 120 stores the behavior information in the storage 130 by compressing the behavior information or by deleting obsolete behavior information. When the first device 100 cannot process the behavior information, the controller 120 can send the behavior to the system server 1000 or other external device, receive the data processed by the external device, and store the processed data in the storage 130.

The storage 130 stores the behavior information together with pre-stored behavior guidelines. The controller 120 generates the health care information by comparing the stored behavior guideline and behavior information.

The behavior guideline may be input directly in the first device 100, or transmitted to the first device 100 from the external device that is connected to the first device 100 and stored in the storage 130. In this case, the first device 100 may further include an input (not shown) or a communication unit (not shown).

The behavior guideline may set according to various types of information corresponding to the user. For example, various behavior guidelines such as prescription medications, exercise plans, food intake plans, and life habits, etc. can be stored in the storage 130.

The controller 120 can notify the user of a behavior guideline and provide information to encourage user compliance with the behavior guideline. For example, when the preset medication administration time or exercise time arrives, the controller 120 outputs a message notifying the user to administer the medication or exercise.

The controller 120 generates the health care information by comparing the behavior guideline stored in the storage 130 with the user behavior information. For example, when prescription administration is stored as the behavior guideline and drug information defined in the prescription is read by the short-range wireless communication reader 110 within a preset time, the controller 120 determines that the prescription has been administered normally according to the behavior guideline and generates the health care information indicating the user is complying with the prescription administration behavior guideline.

When the exercise plan is stored as the behavior guideline and the short-range wireless communication tag in the exercise equipment or the gym door is read by the short-range wireless communication reader 110, the controller 120 determines that the user is complying with the exercise plan and generates the corresponding health care information.

For the food intake, the controller 120 can determine whether the user eats food as usual when the tag of a designated food container is read by the short-range wireless communication reader 110, and record the determination result as the health care information. In addition to the food container, the short-range wireless communication tag may be attached to an entrance or a table of a restaurant to determine the meal time based on the user's presence at the restaurant and/or table. Alternatively, behavior information corresponding to food intake may be generated based on information read by a terminal device of second user such as guardian or caretaker.

With respect to the life habits, the controller 120 can determine when the user goes to sleep when a tag attached to bedding is read by the short-range wireless communication reader 110, and determines that the user is awake when the tag attached to the bedding, an alarm clock, or a bathroom door is read by the short-range wireless communication reader 110, and generates the corresponding health care information.

The controller 120 can generate as the health care information, feedback information generated according to evaluation score and scores based on the comparison of the behavior guideline and the behavior information. The feedback information can include a message notifying a user of the evaluation score, the number of feedbacks, and feedback intensity. For example, when the user conducts a behavior meeting the behavior guideline, a high score or positive points are added to the evaluation score. In this case, a compliment message can be generated as the feedback information. Alternatively, when the behavior of the user does not meet the behavior guideline, this yields a low evaluation score. In this case, a warning message, a number of warning message outputs, a warning sound volume, and a vibration intensity can be generated as the feedback information. That is, the feedback information can include the positive feedback and the negative feedback. The feedback information may be visual information in the form of a graph so that the user can easily check the health care information. Additionally, the feedback may be generated in the form of a request for an emergency rescue in the case that the evaluation score of the user are determined to require such action.

When a preset event arises, the controller 120 outputs a feedback signal according to the feedback information. For example, a positive feedback can output a vibration and music together with a complimentary message so as to feed the current status back to the user. The positive feedback event can occur if the accumulated evaluation score is greater than a certain value or the difference between the accumulated evaluation score and a previous evaluation score is greater than a certain value. Alternatively, negative feedback can output a vibration and a warning sound together with a warning message. The negative feedback event can occur, in one example, when the evaluation score of the health point per part is below a certain value or the difference from a previous evaluation score is greater than a certain value.

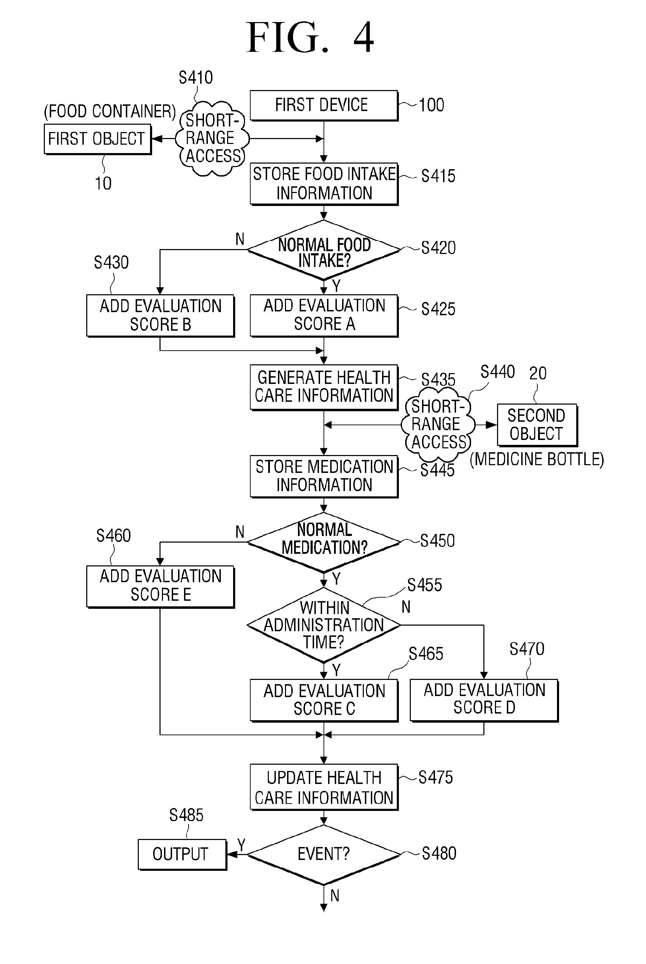

FIG. 4 is a diagram of a method for generating the health care information by calculating the evaluation score. Referring to FIG. 4, when the tag of first object 10 being the food container is detected by the first device (S410), the first device 100 receives the data from the first object 10 and stores the food intake information (S415).

The device compares the received information of the food to the behavior guideline to determine if the food intake is normal (S420). For the normal food intake, the evaluation score 'a' is added to the previous evaluation score (S425). For the abnormal food intake, evaluation score 'b' is added to the evaluation score (S430).

The controller 120 generates and stores the health care information including the evaluation score in the storage 130 (S435). When the health care information of the corresponding user is generated, the controller 120 updates the health care information.

Additionally, when the second object 20 being the medicine bottle approaches (S440) is detected by the first device, the controller 120 generates and stores the medication information in the storage 130 by receiving the information of the second object 20 (S445) .

The controller 120 determines whether the medication is the normal medication by comparing the received medicine information and the medicine information defined by the behavior guideline (S450). When the medication is determined to be abnormal, the controller 120 determines the abnormal medication and adds the evaluation score 'e' (S460). In contrast, when the medication is determined to be normal, the controller 120 determines whether the medication is administered at a proper administration time (S455). If the medication is determined to be administered within the proper administration time, the controller 120 adds evaluation score 'c' (S465). If the medication is determined to be administered outside of the proper administration time, the controller 120 adds the evaluation score 'd' (S470).

The evaluation scores a, b, c, d, and e can be set to appropriate values through repeated experiments. For example, the value 'c' given when the accurate medicine is administered at a correct time can be greater than the value 'd' given when the medicine is administered at the wrong time. The absolute values of 'b' and 'e' given for the abnormal food intake or the abnormal medication can be equal to the absolute values of 'a' and 'c.' While the evaluation score is calculated by adding the positive or negative value or each evaluation score in FIG. 4, the evaluation score may be calculated merely with different positive values. In this case, the differences between 'a,' 'b,' 'c,' 'd,' and 'e' can be increased. Accordingly, when a fixed value is not accumulated during a preset time, the controller 120 can determine that the user's health is not being managed normally.

Upon receiving data by accessing the tags of various external objects, the controller 120 generates the behavior information according to the data and updates the health care information by comparing the behavior information and the behavior guideline (S475). The behavior information may be generated separately based on the data received from the external object, or may be the data itself that is received from the external object.

Alternatively, every time the data is received from the external object, the controller 120 may forward the data itself to the system server 1000 or other devices in real time. Hence, the user's behavior may be detected and the behavior information may be generated externally using the data.

While the behavior information relating to the food intake and the medication is explained in FIG. 4, the behavior information can be generated corresponding to various items such as medication, exercise, and living environment. The behavior information can be generated to correspond to the behavior guideline.

That is, the behavior guideline can include drug information, exercise information, and environment information. The drug information can include information about drug name, effect, efficacy, effect, usage, dosage, ingredients, raw material, period of administration, storage method, cautions of administration, side effects, persons prohibited from taking the drug, persons cautioned about taking the drug, abnormal reaction, survey results after the product release, general directions, interactions with other drugs, and food not to be administered with the drug. The exercise information can include an exercise type, an exercise region, an exercise time, an exercise intensity, an exercise volume, and an exercise speed. The environment information can include temperature, humidity, pressure, and air noxious substances. The food intake information can include information about available food, food to avoid, meal time, intake quantity, nutritional information, and food intake manner.

The health care information stored in the first device 100 can be used for various functions.

As shown in FIG. 4, when a preset event occurs (S480), the first device 100 can output the health care information using a visual message or an audio message (S485). That is, when the first device 100 includes a display (not shown) or a speaker (not shown), the controller 120 can display the health care information through the display or output the health care information as sound using the speaker.

Alternatively, when the first device 100 includes a communication unit, which is not shown in FIG. 4, for communicating with an external device and a preset event takes place, the controller 120 may send the health care information to the external device. Herein, the external device can be the system server 1000 or the second device 200 through the n- th device n of FIG. 1. The second device 200 through the n- th device n can include the user terminal device of a guardian, a user terminal device of a supervisor such as doctor, a hospital server, and an insurance company server.

The event can include various events such as an event where the user inputs a user command to the first device 100, an event where the accumulated health care information meets a preset threshold condition, an event where a transmission request is received from the external device, and an event where a preset time period arrives. When the health care information includes the evaluation score, the threshold condition can specify that the accumulated evaluation score reaches a preset threshold. Also, the event can include a user check signal for checking the evaluation score, or a check request input of the external device. Additionally, the health care information may be a new behavior guideline extracted according to the comparison between the behavior guideline and the behavior information.

While the first device 100 generates the behavior information and then generates and updates the health care information using the behavior information in FIG. 4, the health care information can be generated and updated by the external device such as the system server 1000 and the second device 200 through the n-th device n. In this case, the first device 100 generates the behavior information based on the data received from the objects and then sends the generated behavior information to the external device. For example, the first device 100 can generate the behavior information corresponding to the medication when the medicine bottle information is collected, generate the behavior information of the corresponding food intake when the food container information is collected, and send the generated behavior information to the external device. The external device can compare the behavior guideline defined for the user of the first device 100 with the behavior information, and thus accumulate and manage the score corresponding to the comparison.

When comparing the behavior information and the behavior guideline and determining whether the behavior meets the behavior guideline over a certain period of time, the controller 120 can generate and store a revised behavior guideline in the storage 130. For example, the controller 120 can establish a new behavior guideline to increase exercise volume.

Alternatively, the health care information may be a temporary behavior guideline determined according to the comparison between the behavior guideline and the behavior information. The controller 120 may get the approval of the external device by sending the temporary behavior guideline to the external device. That is, upon receiving the approval information of the temporary behavior guideline from the external device, the controller 120 replaces the behavior guideline stored in the storage 130 with the temporary behavior guideline.

Alternatively, various types of information generated by the first device 100 can be provided to the system server 1000 so that the system server 1000 can offer a user customized health service.

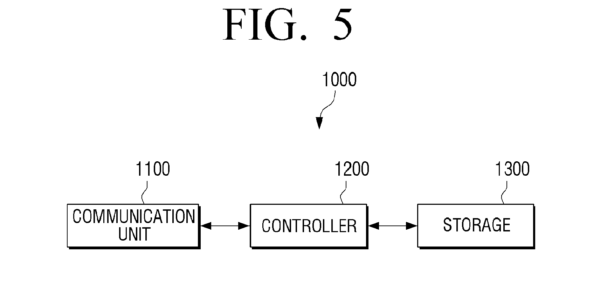

FIG. 5 is a block diagram of the system server 1000. Referring to FIG. 5, the system server 1000 includes a communication unit 1100, a controller 1200, and a storage 1300.

The communication unit 1100 communicates with other devices in the system of FIG. 1. The communication unit 1100 can receive the behavior guideline of the user of the first device 100 from the second device 200. Herein, the user can be a user subscribed to the health management service. That is, the user can subscribe to the service online through a web page provided by the system server 1000 or the second device 200, or offline by submitting an application.

The second device 200 can be a terminal device of the supervisor who is authorized to set the behavior guideline of the user. The supervisor can include a doctor, a nurse, a pharmacist, a health trainer, and a nutritionist.

The controller 1200 controls the communication unit 1100 to forward the behavior guideline received from the second device 200 to the first device 100. Hence, the first device 100 can store the received behavior guideline. The first device 100 can generate the behavior information by collecting data relating to the user's behavior, and send the behavior information to the system server 1000.

When receiving the behavior information through the communication unit 1100, the controller 1200 generates the health care information by comparing the behavior information and the behavior guideline. That is, while the first device 100 itself generates the behavior guideline and the health care information in FIGS. 2 and 4, the system server 1000 generates the health care information in this exemplary embodiment. The health care information generation of the controller 1200 is similar to that of FIG. 4 and thus shall be omitted here.

The controller 1200 stores the generated health care information in the storage 1300. The controller 1200 sends the stored health care information to the second device 200. The second device 200 generates the new behavior guideline by modifying the behavior guideline based on the user's health care information, and sends the generated new behavior guideline to the system server 1000. For example, when the behavior guideline includes the prescription and the supervisor of the second device 200 determines that the user takes medicine steadily according to the prescription, he/she can generate the new behavior guideline by changing the medicine of the prescription, reducing the dosage and/or the administration time, or deleting the prescription.

Upon receiving the new behavior guideline, the controller 1200 controls the communication unit 1100 to forward the new behavior guideline to the first device 100. The first device 100 replaces the pre-stored behavior guideline by the new behavior guideline.

The system server 1000 further includes an input (not shown) for directly receiving and sending the user's basic information to the second device 200. Alternatively, the system server 1000 may receive the user's basic information from the first device 100, that is, the user terminal device and forward the user's basic information to the second device 200.

While the second device 200 generates the behavior guideline in FIG. 5, the first device 100 or the system server 1000 may generate the behavior guideline, and generate and manage the health care information in various implementations.

Hereafter, a user customized health management method for a system according to various exemplary embodiments will be explained.

< The first device generates the behavior guideline >

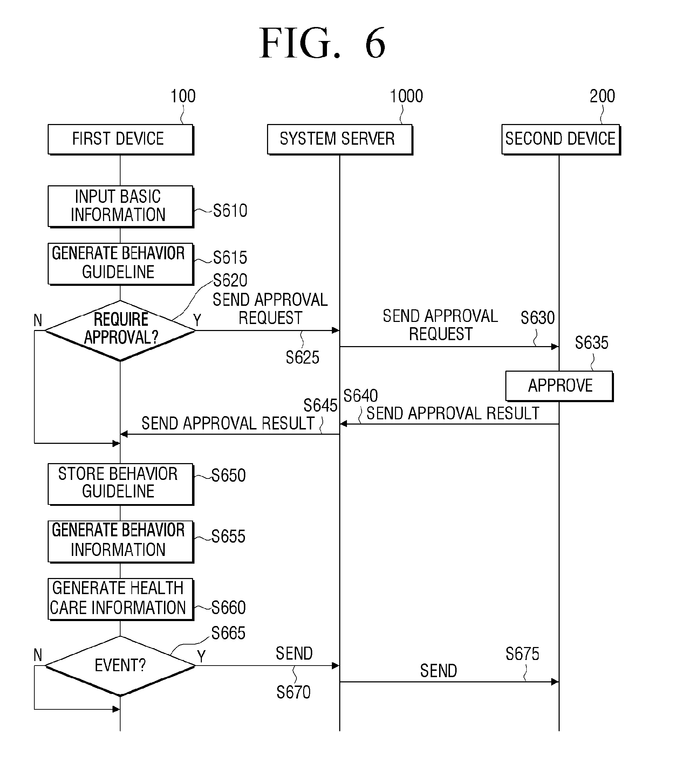

Referring now to FIG. 6, the user's basic information is input to the first device 100 (S610). The first device 100 can be implemented as the user terminal device of the user as described above. The basic information can include identification information such as a user's name, user ID, and resident registration number, characteristic information such as gender, age, weight, and height, and special information such as user medical history, therapy record, family anamnesis, physical constitution, and allergies.

A user such as a patient may directly input the basic information to the first device 100, or information input by the doctor or other expert may be sent and input to the first device 100. Alternatively, the basic information may be input using any suitable form of communication, for example short-range wireless communication by reading a tag of an external object such as an identification card or service membership card containing the user's basic information using the first device 100.

The first device 100 can generate the behavior guideline based on the received information (S615).

The behavior guideline can include various guidelines corresponding to prescription medications, food intake manner, healthy diet, dietary menu, exercise plan, and daily diet schedule. The behavior guideline may be divided into a guideline requiring approval, and a guideline not requiring approval, that is, into a first behavior guideline effective upon the approval from another person such a doctor or supervisor and a second behavior guideline without the approval.

As an example of the behavior guideline requiring the approval, the prescription may be input to the first device 100 by downloading information prescribed by the doctor from the external device or by using a barcode or a QR code printed on the prescription, or an NFC tag attached to the prescription. The user may also view the prescription label and input the prescription information on the label to generate the behavior guideline. Such a behavior guideline can be set as the temporary behavior guideline.

As such, when the user can input the doctor's prescription user to the first device 100, the external approval can be required to verify the prescription. Upon the external approval for the temporary behavior guideline, the generated behavior guideline is replaced by the temporary behavior guideline.

Alternatively, the behavior guideline that does not require approval can be input in various manners as described above. The behavior guideline without the approval may be automatically generated by the first device 100. For example, as for the dietary menu or the exercise plan, the first device 100 can calculate a standard body weight and an obesity index using the weight and height information input by the user, and automatically calculate a weight loss goal corresponding to the obesity index. Hence, it is possible to determine a daily recommended caloric intake, an exercise volume a day, a recommended menu, and a recommended exercise volume corresponding to the weight loss goal from a pre-stored database. The database, which records optimal values obtained through a plurality of repeated experiments, can be pre- stored in the first device 100.

Other behavior guidelines may be defined and input by the user in person using the first device 100, or may be defined using another terminal device of the user, sent to the first device 100, and stored in the first device 100.

When the generated behavior guideline requires the approval (S620:Y), the first device 100 sends an approval request including the basic information and the behavior guideline to the system server 1000 (S625). While the behavior guideline corresponding to the prescription medication information may require the approval, various behavior guidelines such as exercise and diet may not require the approval.

Upon receiving the approval request from the first device 100, the system server 1000 resends the approval request to the second device 200 matching the user of the first device 100 (S630). The system server 1000 can match the supervisor to the user subscribed to the health management service, and store an Internet Protocol (IP) address, an e-mail address, and a phone number of the second device 200 of the matched supervisor. Hence, when the subscribed user logs on and sends the approval request, the system server 1000 searches for the second device 200 by matching the user among the pre-stored information of the second devices, and sends the approval request to the matching second device 200.

The second device 200, upon receiving the approval request, displays a UI screen for the approval. Hence, the supervisor can check the user's basic information and behavior guideline through the UI screen and thus grant the approval (S635).

Upon the approval, the second device 200 sends an approval result to the system server 1000 (S640). The system server 1000 forwards the approval result to the first device 100 (S645).

When the approval is received, the first device 100 stores the behavior guideline (S650). Alternatively, when the approval is unnecessary (S620:N), the first device 100 stores the behavior guideline regardless of the approval (S650). If no approval result is received over a preset time, the first device 100 can resend the approval request for a preset number of times. Alternatively, when the approval fails, the first device 100 may resend the approval request by regenerating the behavior guideline, or requesting that the second device 200 to generate and send a behavior guideline that is approved. For example, the doctor using the second device may send a new approved behavior guideline in place of the behavior guideline that was received for approval.

When information about the user's behavior is collected with the behavior guideline stored, the first device 100 generates the behavior information based on the collected information (S655). The generation of the behavior information can be fulfilled in various manners as discussed above.

The first device 100 can generate the health care information by comparing the behavior information and the behavior guideline (S660). The health care information can be the feedback information generated according to the evaluation score determined by the behavior information, the new behavior guideline extracted by comparing the behavior guideline and the behavior information, or the temporary behavior guideline.

When the health care information is the new behavior guideline, the pre-stored behavior guideline is replaced by the new behavior guideline. When the health care information is the temporary behavior guideline and the external approval is received, the pervious behavior guideline is replaced by the temporary behavior guideline. As such, the behavior guideline can be changed dynamically according to the user's behavior.

When the health care information is stored and a preset event occurs (S665), the first device 100 sends the health care information to the system server 1000 (S670). The system server 1000 forwards the health care information to the second device 200 (S675).

While the first device 100 generates the behavior guideline and also the health care information for the health management in FIG. 6, the system server 1000 may generate and manage the health care information. In this case, the first device 100 can display various guidance messages according to the behavior guideline. When collecting the data about the user's behavior, the first device 100 can generate the behavior information based on the collected data and send the behavior information to the system server 1000. The behavior guideline generation of the first device 100 and the generation and the management of the health care information of the system server 1000 are substantially similar to those explained in FIG. 6 and shall be omitted here.

An entity for receiving the user's basic information, an entity for generating the behavior guideline, and an entity for generating the health care information can differ according to implementations.

< The system server generates the behavior guideline >

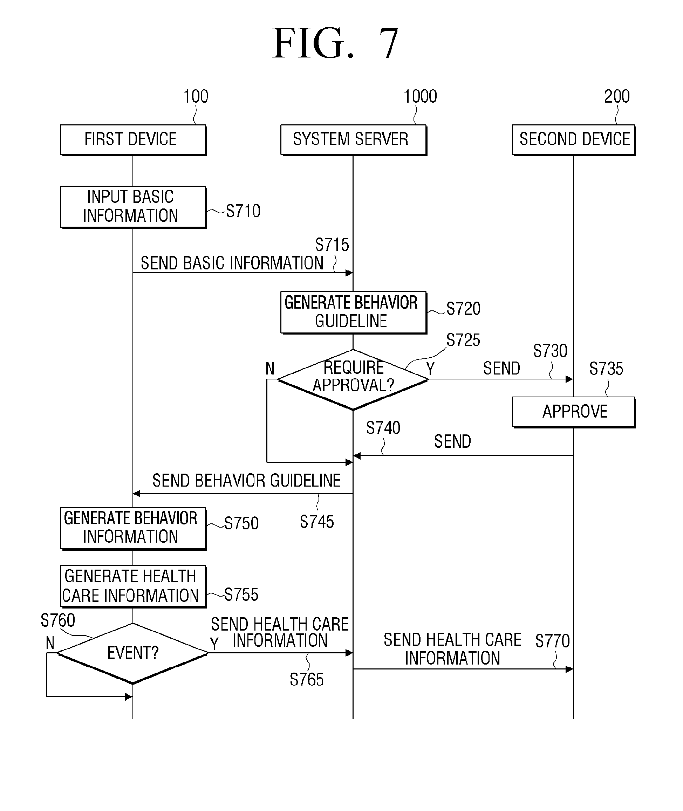

FIG. 7 is a timing diagram of a user customized health management method for a system according to another exemplary embodiment.

Referring to FIG. 7, the user inputs the user basic information to the first device 100 being his/her terminal device (S710), and the input basic information is transmitted to the system server 1000 (S715). In this case, the user can obtain his/her account by requesting the service subscription from the system server 1000 online or offline. The user can log on to the system server 1000 by inputting his/her ID and password. To open an account, the user may input information or provide various information that is modified by the user after the logon. This information can be provided to the system server 1000 as the basic information.

The system server 1000 generates the behavior guideline using the received basic information (S720). The behavior guideline can vary as stated earlier. The generation of the behavior guideline has been explained in FIG. 6 and thus shall be omitted here.

When generating the behavior guideline, the system server 1000 determines whether the behavior guideline requires the approval (S725). When the approval is required (S725:Y), the system server 1000 sends the approval request including the basic information and the behavior guideline of the user to the second device 200 (S730).

Upon receiving the approval request, the second device 200 displays an approval screen including the user's basic information and behavior guideline so that the supervisor can grant the approval. Hence, upon the approval (S735), the second device 200 sends the approval result to the system server 1000 (S740). When the approval result is received or the behavior guideline does not need the approval, the system server 1000 sends the behavior guideline to the first device 100 (S745).

The first device 100 stores the received behavior guideline and generates the behavior information using the collected data (S750).

The first device 100 generates the health care information by comparing the generated behavior information and the behavior guideline (S755). When a particular event occurs (S760:Y), the first device 100 sends the health care information to the system server 1000 (S765). The system server 1000 forwards the health care information received from the first device 100 to the second device 200 (S770).

The behavior information generation method or the health care information generation method, and the event type have already been described in detail and shall be omitted here.

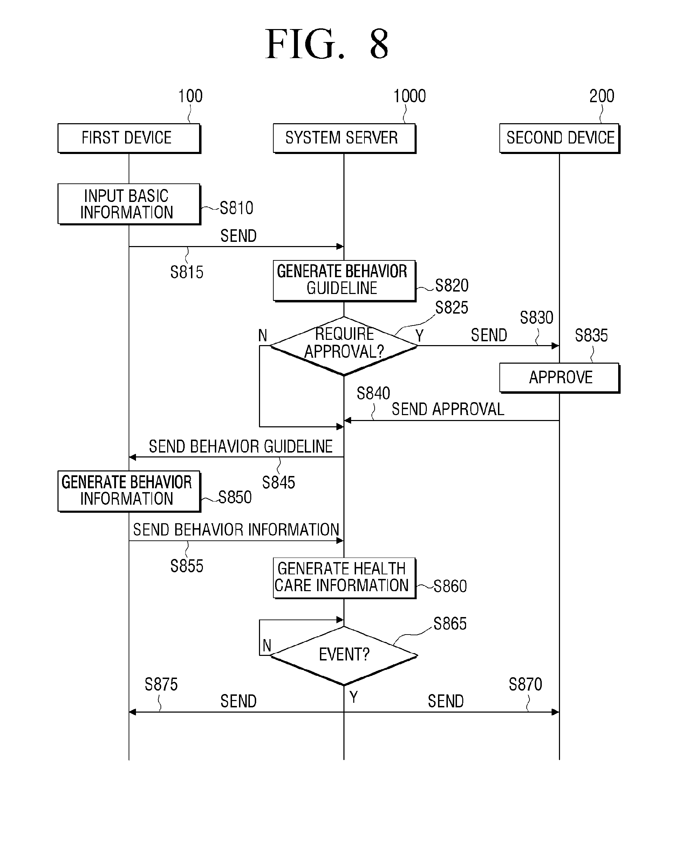

FIG. 8 illustrates the generation of both the behavior guideline and the health care information in the system server 1000.

Referring to FIG. 8, the user inputs the user basic information to the first device 100 (S810), and the input basic information is transmitted to the system server 1000 (S815). The system server 1000 generates the behavior guideline using the received basic information (S820). When the approval is required (S825), the system server 1000 sends the approval request to the second device 200 (S830) and receives the approval (S835 and S840).

When the approval is completed or the behavior guideline is determined without approval, the system server 1000 stores the determined behavior guideline in the account given to the user of the first device 100.

The system server 1000 sends the generated behavior guideline to the first device 100 (S845). Upon receiving the behavior guideline, the first device 100 outputs adequate message including instructions corresponding to the behavior guideline such as an indication of a time and/or a place of the behavior guideline so that the user can behave to the behavior guideline. A type of the output message shall be explained. If the user does not need to know the behavior guideline, the transmission of the behavior guideline to the first device 100 may be omitted.

The first device 100 generates the user's behavior information by collecting data about the user's behavior (S850). The generated behavior information is transmitted to the system server 1000 (S855).

The system server 1000 generates the health care information by comparing the received behavior information and the pre-stored behavior guideline of the corresponding user (S860).

When a preset event takes place (S865), the system server 1000 sends the health care information to the first device 100 and the second device 200 respectively (S870 and S875).

In some of the above described embodiments, the behavior guideline may be generated by the first device 100 or the system server 1000, whereas the behavior guideline requiring the confirmation of the expert needs to be approved by the external device (i.e., the second device). In this respect, the expert being the supervisor can generate the behavior guideline using his/her terminal device. Hereafter, the behavior guideline generation in the second device is explained.

< The second device generates the behavior guideline >

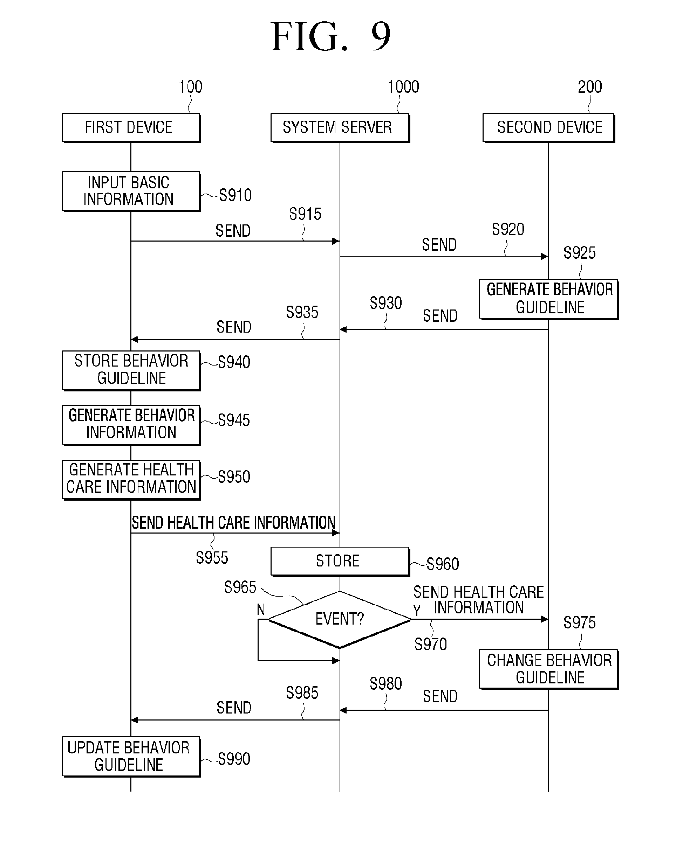

Referring to FIG. 9, when the basic information is input (S910), the first device 100 sends the basic information to the system server 1000 (S915). The system server 1000 searches its pre-stored database for the second device 200 being the supervisor's terminal device matching the first device 100. When the system server 1000 is implemented using a hospital server, the system server 1000 may contain a database including information about terminal devices of doctors, nurses, nutritionists, and rehabilitation trainers working in or cooperatively related to the hospital. Hence, the system server 1000 can select the supervisor matching the user's basic information using the database. For example, when the user subscribes to the health management service in order to cure his/her disease, the system server 1000 can match a medical specialist of the corresponding disease to the corresponding user, select the medical specialist as the supervisor for the user, and record this information in the database. Alternatively, when the user receives the rehabilitation therapy, the system server 1000 can select the rehabilitation trainer as the supervisor and match him/her with the user. Additionally, the system server 1000 can match the supervisor by collectively considering the user's age, gender, nationality, status, and service subscription purpose. The supervisor may be matched manually by the administrator who manages the system server 1000, or automatically according matching algorithm that matches the basic information of the user with an appropriate supervisor.

When detecting the second device 200, the system server 1000 sends the user's basic information to the second device 200 (S920).

The second device 200 generates the behavior guideline based on the generated basic information (S925). The second device 200 sends the generated behavior guideline to the system server 1000 (S930). The system server 1000 forwards the behavior guideline to the first device 100 (S935).

The first device 100 stores the received behavior guideline (S940). The first device 100 can utilize the behavior guideline in various ways. For example, when the behavior guideline defines the administration time and the medication information, the first device 100 can notify the user to take the medicine by displaying the medicine information at a fixed time. When the exercise time arrives, the first device 100 can notify the user to exercise by outputting an alarm signal.

The first device 100 generates the behavior information by collecting the user's behavior information according to various collection methods (S945).

Hence, the first device 100 generates the health care information by comparing the generated behavior information with the behavior guideline (S950). The health care information can be generated diversely. That is, as mentioned in FIG. 4, the health care information can include the evaluation score accumulated according to the conformity of the behavior information and the behavior guideline of the user. The health care information may also include the feedback information corresponding to the evaluation score. The evaluation score calculation and the feedback information have been described in detail and shall be omitted here.

The first device 100 sends the generated health care information to the system server 1000 (S955). The system server 1000 stores the received health care information (S960). At this time, when a preset event occurs (S965), the system server 1000 sends the health care information to the second device 200 (S970).

Herein, the event can vary as stated earlier. For example, when the evaluation score exceeds a first threshold or falls below a second threshold, the health care information can be transmitted. When a user's service subscription period or a preset test period expires, the system server 1000 can send the health care information.

Alternatively, the system server 1000, when determining that the user's status reaches the goal, may send the health care information. When the user of the first device 100, the supervisor of the second device 200, the administrator of the system server 1000, and other guardian requests to change the behavior guideline, the system server 1000 may send the health care information to the second device 200.

The second device 200 can check the user's health care information, predict the user's status change, and change the behavior guideline (S975). The supervisor of the second device 200 can, when determining that the user successfully lives up to the behavior guideline, input an enhanced behavior guideline in order to achieve better results for the user. In contrast, when the user cannot keep up with the behavior guideline, the supervisor of the second device 200 can input moderate behavior guideline so that the user can keep up with the behavior guideline.

The behavior guideline can be divided into the long-term behavior guideline and the short-term behavior guideline as described above. The long-term behavior guideline changes according to a preset algorithm based on the cumulative score, the behavior information, personal health information, and medical information. For example, the long-term behavior guideline can include a medication type, an administration time, a dosage, an optimal exercise type, an exercise time, an exercise volume, a food intake amount, a meal time, an optimal food type, an optimal bowl volume, a bowl type, a sleeping time, a bedtime, a wake-up time, and a sleeping environment. The supervisor can modify these various long-term behavior guidelines according to the status of the user. The short-term behavior guideline can be modified collaterally according to the long-term behavior guideline.

Thus, when the new behavior guideline modified from the previous behavior guideline is input in the second device 200 (S975), the second device 200 sends the behavior guideline to the system server 1000 (S980). The system server 1000 forwards the behavior guideline to the first device 100 (S985). The first device 100 updates the behavior guideline with the received new behavior guideline (S990).

While the user's basic information is input through the first device 100 and forwarded to the second device 200 via the system server 1000 in FIG. 9, the user's basic information may be input directly to the second device 200 in case of an offline contract. For example, when the user consults his/her doctor at a hospital, the user's basic information obtained in the medical treatment can be input directly to the second device 200 managed by the doctor or the nurse.

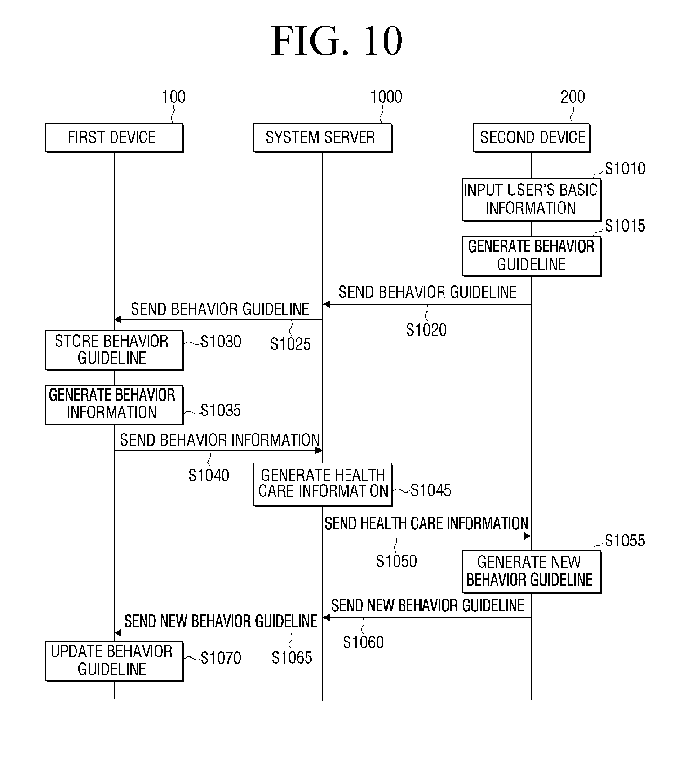

FIG. 10 depicts the direct input of the user's basic information to the second device. Referring to FIG. 10, the user's basic information is input directly to the second device 200 (S1010), and his/her behavior guideline is generated together (S1015).

The second device 200 sends the generated behavior guideline to the system server 1000 (S1020). The system server 1000 forwards the behavior guideline to the first device 100 of the user who took the medical treatment (S 1025).

The first device 100 stores the behavior guideline (S1030) to induce the user's appropriate behavior using the stored behavior guideline in various manners.

The first device 100 generates the behavior information of the user (S1035) and sends the behavior information to the system server 1000 (S1040). The system server 1000 generates the health care information by comparing the received behavior information and the behavior guideline (S1045).

The system server 1000 sends the generated health care information to the second device 200 (S1050). The health care information can be transmitted to the second device 200 when the second device 200 requests the transmission or a preset transmission cycle arrives. The health care information may be transmitted to the first device 100, which is not depicted in FIG. 10.

Upon receiving the health care information, the second device 200 displays a behavior guideline input screen including the health care information. The supervisor generates the new behavior guideline by modifying the behavior guideline of the user of the first device 100 through the behavior guideline input screen (S1055).

The second device 200 sends the generated new behavior guideline to the system server 1000 (S1060), and the system server 1000 forwards the received new behavior guideline to the first device 100 (S1065).

The first device 100, upon receiving the new behavior guideline, updates the previous behavior guideline (S1070). The first device 100 and the system server 1000 manage the user's behavior according to the newly updated behavior guideline.

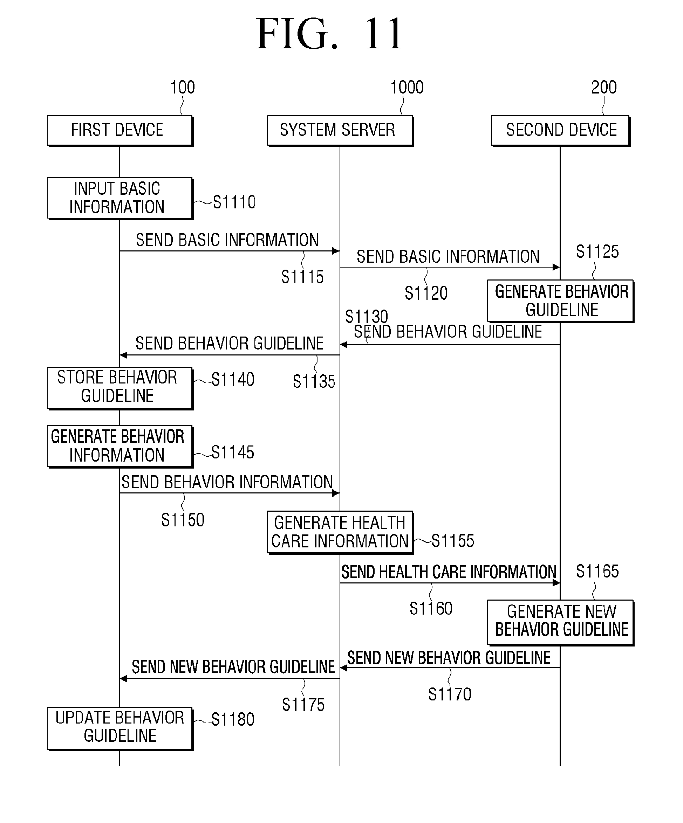

FIG. 11 illustrates operations of a system according to another exemplary embodiment. Referring to FIG. 11, the user's basic information is input to the first device 100 (S1110).

The input basic information is transmitted to the second device 200 via the system server 1000 (S1115 and S1120). The second device 200 generates the behavior guideline based on the user's basic information (S1125), and provides the generated behavior guideline to the first device 100 via the system server 1000 (S1130 and S1135). The system server 1000 stores the behavior guideline in a storage space allocated for the user of the first device 100.

The first device 100 stores the received behavior guideline (S1140), generates the behavior information (S1145), and sends the behavior information to the system server 1000 (S1150). The system server 1000 generates the health care information by comparing the received behavior information and the behavior guideline stored for the user of the first device 100 (S1155).

The system server 1000 sends the generated health care information to the second device 200 (S1160). The new behavior guideline modified from the behavior guideline based on the health care information that is input to the second device 200 (S1165). The second device 200 sends the new behavior guideline to the system server 1000 (S1170).

The system server 1000 forwards the new behavior guideline to the first device 100 (S1175). The first device 100 updates the behavior guideline according to the received new behavior guideline (S1180).

As such, the user's basic information input or the behavior guideline generation can be carried out variously in the first device 100, the system server 1000, and the second device 200. In particular, the user's basic information can be manually input in the device of the user, the system administrator, or the supervisor (i.e., the doctor). In this case, when simple identification information such as user's resident registration number, previously allocated management number, or user ID is input, necessary information may be collected by automatically linking to user's personal health information stored in the system server 1000 or a separate external server. For example, when the user inputs the resident registration number, the system server 1000 can collect various information such as user's therapy record, medical history, family anamnesis, and insurance payment record from a one or more databases located in the server, a medical insurance corporation server, a hospital server, and an insurance company server, and utilize the information for generation of the behavior guideline.

In some embodiments, the behavior guideline is generated by and received from the external device, that is, the system server 1000 or the second device 200, rather than the first device 100. In this case, the first device 100 can receive the behavior guideline together with various additional information from the external device. For example, the additional information can include general information useful for the health care, an advertisement, insurance data, information about the positive effects of adhering to the behavior guideline, and information about the negative effects of not adhering to the behavior guideline. The first device 100 can store and use the behavior guideline and the additional information. When receiving such new information, the first device 100 updates the previous information with the new information.

The health care information can be also generated and managed by the first device 100 or the system server 1000. While the health care information generation in the second device 200 is not illustrated in the aforementioned embodiments, the second device 200 may generate and manage the health care information in some cases.

< Association with other device >

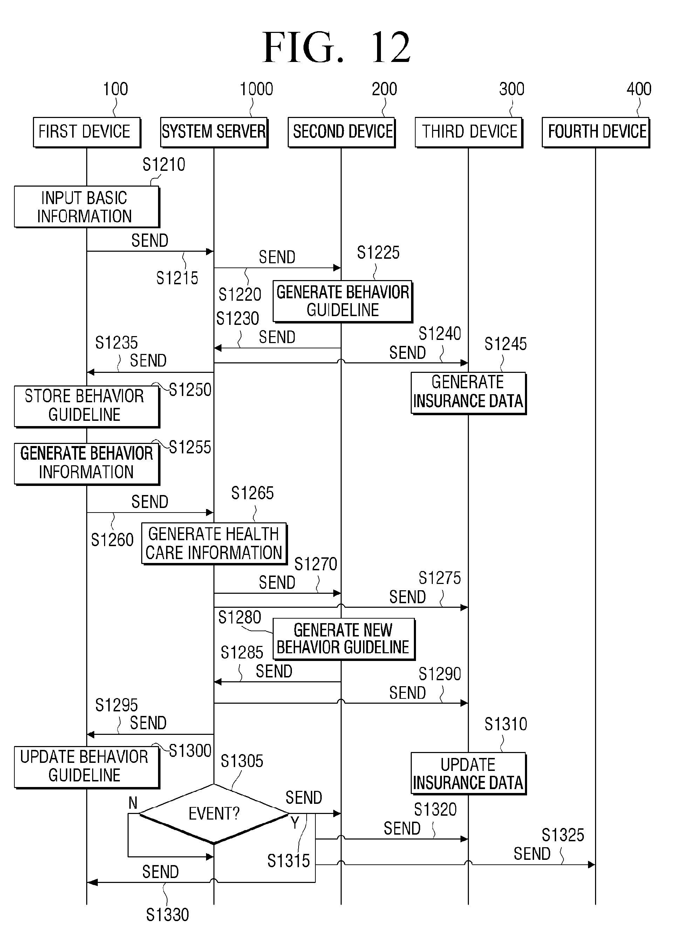

FIG. 12 depicts the health management service support from other devices in addition to the first device 100, the system server 1000, and the second device 200.

Referring to FIG. 12, when the basic information is input to the first device 100 (S1210), the first device 100 sends the basic information to the system server 1000 (S1215).

The basic information can further include information of the third and fourth devices 300 and 400 in addition to various user information. The third and fourth devices 300 and 400 can employ a server or a terminal device of another person of various interests. In FIG. 12, it is assumed that the third device 300 is implemented using the insurance company server and the fourth device 400 is implemented using the guardian terminal device.

The system server 1000 forwards the basic information to the second device 200 (S1220).

The second device 200 generates the behavior guideline based on the received basic information (S1225). The second device 200 sends the generated behavior guideline to the system server 1000 (S1230).

The system server 1000 forwards the received behavior guideline to the first device 100 and the third device 300 (S1235 and S1240). If necessary, the system server 1000 can also send the behavior guideline to the fourth device 400, which is not depicted in FIG. 12.

Upon receiving the behavior guideline, the third device 300 can generate insurance data based on the received behavior guideline (S1245). That is, the third device 300 can calculate an insurance fee, an insurance benefit, an insurance period based on the various information of the user. The calculated insurance data can be transmitted to the first device 100 or the fourth device 400 and used to make an insurance contract or to renew the insurance.

To share information with the insurance company, the operator of the third device 300, that is, the insurance company can make an arrangement with the administrator of the system server 1000. That is, the present general inventive concept can be realized as a Business to Business (B2B) model based on the arrangement between businesses, to be explained.

Meanwhile, upon receiving the behavior guideline, the first device 100 stores the received behavior guideline (S1250) and generates the behavior information (S1255) . The generated behavior information is transmitted to the system server 1000 (S1260) .

The system server 1000 generates the health care information by comparing the behavior information and the behavior guideline (S1265).

The system server 1000 sends the generated health care information to the second device 200 and the third device 300 respectively (S1270 and S1275).

The second device 200 generates the new behavior guideline based on the user's health care information (S1280). The new behavior guideline is sent to the system server 1000 (S1285).

The system server 1000 sends the new behavior guideline to the first device 100 and the third device 300 (S1290 and S1295).

The first device 100 updates the behavior guideline using the new behavior guideline (S1300).

The third device 300, when receiving the new behavior guideline, updates the insurance data based on at least one of the health care information and the new behavior guideline of the user (S1310). The insurance data can be updated manually according to the direct input of the user of the third device 300, or automatically according to a preset insurance fee calculation algorithm. For example, when the evaluation score of the health care information is high, the insurance fee can be lowered in proportion to the evaluation score. When the evaluation score is low, the insurance fee can be raised.

When a preset event arises (S1305), the system server 1000 can send various information such as previous behavior guideline, new behavior guideline, user basic information, and health care information to the first through fourth devices respectively (S1315, S1320, S1325, S1330).

In FIG. 12, various lifestyle management services, consumer services, as well as health management can be performed together by considering the behavior guideline created for the user and the user's actual behavior.

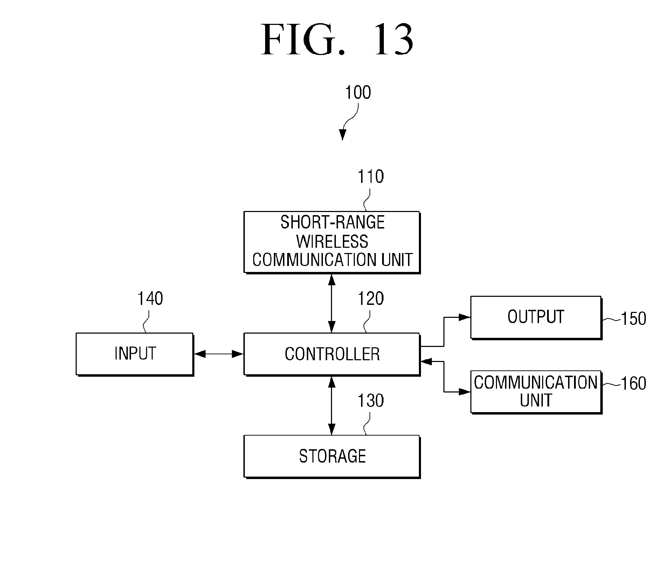

FIG. 13 is a block diagram of the first device 100 according to an exemplary embodiment.

Referring to FIG. 13, the first device 100 includes a short-range wireless communication unit 110, a controller 120, a storage 130 (e.g. storage unit), an input 140, an output 150, and a communication unit 160.

The input 140 can include various input means such as touch screen, touchpad, button, remote control signal interface, keyboard, mouse, and joystick. Hence, the input 140 receives and forwards the user's basic information or various user commands to the controller 120.

The controller 120 operates according to the command input through the input 140. The controller 120 includes a system memory, a main CPU, an image processor, and various interfaces. The controller 120 can include an interface connected to the storage 130, a network interface connected to the communication unit 160, and an interface connected to other port. A detailed structure and operations of the controller 120 shall be explained further.

The controller 120 generates the behavior information using an application stored in the storage 130, and stores the generated behavior information in the storage 130. The communication unit 160 transmits the behavior information stored in the storage 130 to the system server 1000. According to the execution of the application, the controller 120 can offer the user customized health management service according to various aspects of exemplary embodiments. Hereafter, the operations of the controller 120 according to an aspect of an exemplary embodiment will be explained.

When the user's basic information is input to the first device 100, the controller 120 generates a User Interface (UI) screen by driving the embedded image processor and displays the UI screen through the output 150. The image processor can perform various image processes such as graphic rendering and scaling.

The output 150 can include a display unit and a speaker. The output 150 displays the UI provided from the controller 120 through the display unit. The user can input the basic information using the input 140 on the UI. The controller 120 can send the input basic information to the system server 1000 or other external device. As mentioned above, the basic information can include the identification information such as user's name, user ID, and resident registration number, and management number for identifying the user, the characteristic information such as height, weight, age, gender, blood type of the user, and the special information such as user therapy record, medical history, family anamnesis, physical constitution, life habit, and exercise history. The medical information such as therapy record, medical history, and family anamnesis may be referred to as health information. The basic information may be sensed by a separate biometric sensor or received from an external device, rather than being input by the user in person.

When the first device 100 directly generates and manages the behavior guideline and the health care information, the controller 120 generates various behavior guidelines using the user's input basic information. The behavior guidelines can include various information such as exercise information, food intake information, medication information, and living environment information. The behavior guidelines can include the first behavior guideline requiring the external approval and the second behavior guideline without the external approval. The behavior guidelines may include the long-term behavior guidelines and the short-term behavior guidelines.

An example of behavior guidelines generated based on the user's basic information is shown in the following table.

[Table 1]

| Age | Height | Weight | Recommended behavior guideline |
|---|---|---|---|
| 10~15 | ~140 | ~50kg | Take calcium tablet twice a day. Take vitamin once a day. Jump rope 200 times a day. |
| 10~15 | 140~150 | 50~60kg | Take calcium tablet twice a day. Jump rope 100 times a day. |
| 10~15 | 160~170 | 60~70kg | Take vitamin once a day. Jog three times a week. |
| 10~15 | 170~180~ | 70~80kg | Jog three times a week (each 1 hour). Low-salt diet. |
| 10~15 | 180~ | 80kg~ | Exercise more than 3 times a week without placing strain on the knees (recommended: walking; not recommended: running, rope skipping). Maintain a low-calorie diet. Take vitamin once a day. |
| 16~10 | ... | ... | ... |
| ... | ... | ... | ... |

In Table 1, the age, the height, and the weight correspond to the user's basic information. The recommended behavior guideline indicates an optimal behavior guideline according to each element of the basic information of user. Using the database of FIG. 1, the controller 120 can automatically define the behavior guideline according to the user's basic information.

The recommended behavior guidelines for the age range between 10 ~ 15 and the height and the weight are individually set in Table 1 for simplified explanation. However, a plurality of weight ranges can be classified based on the height range within the same age range and the recommended behavior guideline can vary per weight range.

The recommended behavior guideline can be subdivided by adding various items such as gender, nationality, region, and heart rate to the basic information.

The type and the generation of the behavior guideline have been described already and shall be omitted here.

The controller 120 stores the generated behavior guideline in the storage 130. When the behavior information is generated, the controller 120 generates the health care information by comparing the behavior information and the behavior guideline. The health care information can calculate and record the evaluation score based on the comparison result of the behavior information and the behavior guideline as aforementioned. For example, the health care information can be generated as follows.

[Table 2]

| Exercise Guideline | Actual Exercise | Reference Heart Rate | Actual Heart Rate | Cumulative Score | Feedback Type | Feedback Information | Modified Exercise Guideline |
|---|---|---|---|---|---|---|---|
| 3 times a week | 3 times a week | 60 ~ 70 | 65 | 10 | Popup Message | Good for you! Increase exercise intensity? | 4 times a week |
| 3 times a week | Once a week | 60 ~ 70 | 77 | 6 | Popup Message | Heart is beating fast. Please close your eyes and take a deep breath. | |
| 3 times a week | 5 times a week | 60 ~ 70 | 0 | 10 | Alarm & Emergency Rescue Request | Emergency Cardiac Care Required | |

In Table 2, when the behavior guideline is set to 3 times a week, the behavior information, that is, the actual exercise that is recorded is set to 3 times, once, and 5 times a week. The exercise guideline of Table 2 is the behavior guideline adjustable by the user, and a reference heartbeat or an actual heartbeat corresponds to a health index of the user which is measured and not controlled by the user. Another example of the health index may be blood pressure or blood sugar.

The cumulative score can be automatically calculated by the device. When the activity exceeds the number of times defined by the behavior guideline, the cumulative score of 10 is applied. The cumulative score of 6 is applied when the user exercises only once a week. As the basic information includes the user's heart rate, the feedback information content and the feedback type are determined according to heart rate information. The heart rate information can be sensed by a bio-signal sensor connected to or embedded in the first device 100. Hence, the behavior can be monitored based on the behavior guideline. If an abnormal symptom of the user is detected, the user may be provided with emergency care.

Based on a database containing information similar to that of Table 1 and Table 2, the controller 120 can generate the behavior guideline and the health care information. When the generated behavior guideline requires the external approval, the request is sent to the external device to request the approval, which has been explained above and is omitted here.

When the first device 100 generates the behavior guideline and the system server 1000 generates the health care information, the controller 120 can send the generated behavior guideline to the system server 1000.

Meanwhile, when the system server 1000 or the second device 200 generates the behavior guideline, the controller 120 receives the behavior guideline through the communication unit 160 and stores the behavior guideline in the storage 130. Hence, the controller 120 can generate the health care information by comparing the behavior guideline and the behavior information, and store the generated health care information in the storage 130.

When the system server 1000 generates and manages the health care information, the controller 120 may send only the behavior information to the system server 1000 and be controlled by the system server 1000.

As above, the operations of the components of the first device 100 can differ according to the implementation and have been described in the exemplary embodiments, and thus shall be omitted here.

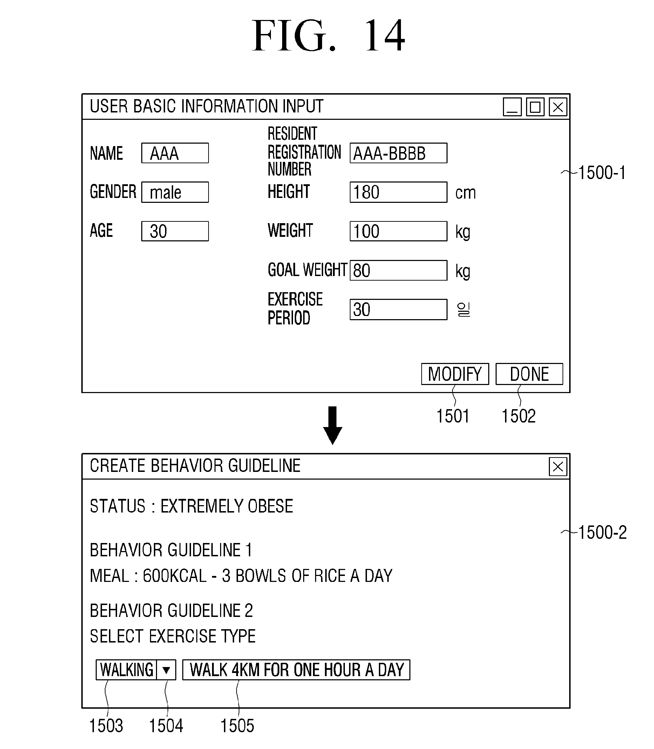

FIG. 14 depicts an input screen for inputting the user's basic information. When the user basic information is input to the first device 100 and the first device 100 generates the behavior information, the first device 100 displays the screen of FIG. 14. Referring to FIG. 14, the input screen 1500-1 displays an input region for inputting various items such as name, gender, age, resident registration number, height, weight, goal weight, and exercise duration, and various menu items 1501 and 1502 for modifying or ending the input. The user can input the basic information by touching the input region and entering numbers or characters in person, and complete the basic information input by selecting the end menu item 1502.

When the user finishes the basic information input, the input basic information is stored and the behavior guideline is automatically generated according to the stored input information. Accordingly, a behavior guideline generation screen 1500-2 is displayed. The behavior guideline generation screen 1500-2 displays the current user status and various behavior guidelines.

In FIG. 14, the diet guideline and the exercise guideline are generated and displayed. All of the behavior guidelines may be automatically generated as stated earlier, whereas some behavior guidelines may be selected by the user. For example, the first behavior guideline is automatically set, and the exercise guideline being the second behavior guideline can be selected by the user as shown in FIG. 14. Thus, the behavior guideline generation screen 1500-2 displays an exercise information region 1503 showing the exercise type, a selection button 1504 for changing the exercise type, and an exercise volume region 1505 indicating the exercise volume based on the selected exercise type. When the selection button 1504 is selected, a list of the selectable exercise types is opened under the exercise information region 1503. When the user selects the walking in the list, the appropriate walking distance is displayed in the exercise volume region 1505.

The screen of FIG. 14 can be displayed in the device which inputs the user's basic information. That is, one of the first device 100, the system server 1000, and the second device 200 can display the screen of FIG. 14 so as to input the basic information.

The diet guideline or the exercise guideline of FIG. 14 can generate the behavior guideline without the external approval. However, the user or the first device 100 cannot arbitrarily generate the behavior guideline for the medication. In this case, the second device 200 can generate and send the behavior guideline to the first device 100 as shown in FIGS. 9, 10 and 11.

The input screen of FIG. 14 can be the screen in a webpage accessed by the user to subscribe to the user customized health management service operated by the administrator of the system server 1000. For example, when the user accesses the web page of the administrator online and applies for the service, a screen for inputting the user's basic information similar to the basic information input screen 1500-1 of FIG. 14 can be displayed in the process of subscribing to the service.

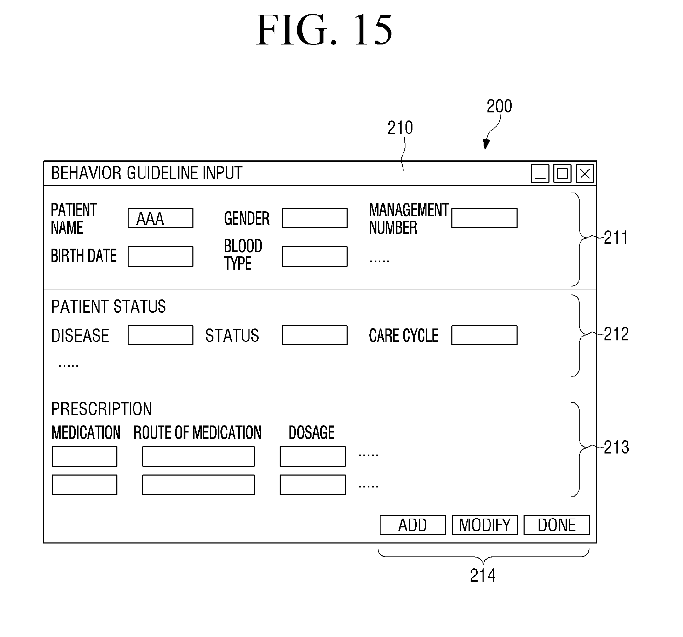

FIG. 15 depicts an input screen 210 displayed to generate the behavior guideline in the second device 200.

The input screen 210 displays a region 211 for inputting the user's identification information and characteristic information, a region 212 for inputting the special information including the user's health information, a region 213 for inputting the user prescription, and various menu items 214 for modifying, adding, or ending the information input.

The supervisor using the second device 200 inputs necessary information in each region. In detail, the supervisor can enter the medicine to administer, the route of the administration, and the dosage. Hence, the user's behavior guideline is generated.

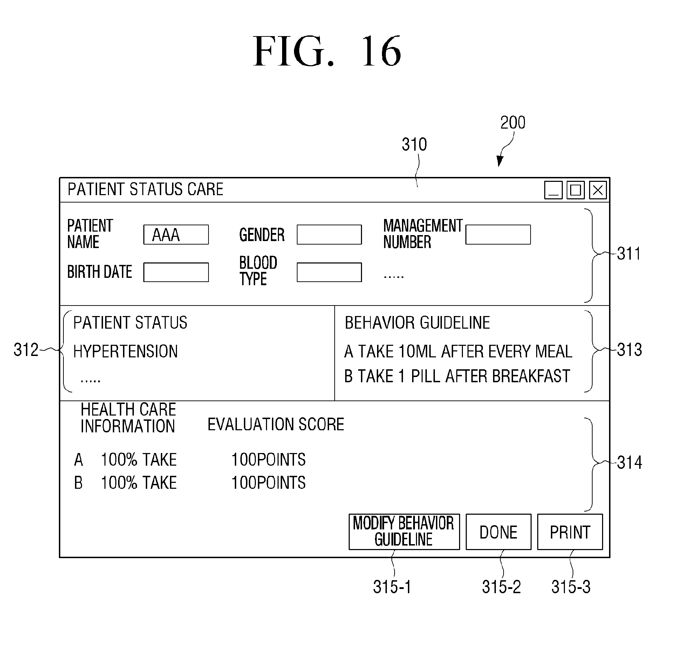

FIG. 16 depicts an input screen for inputting the new behavior guideline by modifying the user behavior guideline.

Referring to FIG. 16, the input screen 310 displayed in the second device 200 displays a region 311 for displaying the user's identification information and characteristic information, a region 312 for inputting the special information including the user health information, a region 313 for displaying the behavior guideline information generated by the user in advance, a region 314 for displaying the health care information generated by comparing the user's behavior information and behavior guideline, a menu item 315-1 for modifying the behavior guideline, a menu item 315-2 for completion of the entry, and a menu item 315-3 for printing.

In FIG. 16, the evaluation score calculated per drug is displayed as the health care information while the behavior guideline defines the administration route. From the input screen of FIG. 16, the supervisor can determine whether the user takes good care of his/her health and whether to modify the behavior guideline. When the supervisor selects the behavior guideline modify menu item 315-1 on the input screen 310 of FIG. 16, the input screen 210 of FIG. 15 is displayed again and the supervisor can modify the behavior guideline on the input screen 210 of FIG. 15.

Besides the user's behavior information, biological information indicating the actual body condition of the user can be sensed and provided together. For example, the biological information such as blood pressure, blood sugar, body temperature, and heart rate can be provided. The biological information can be also displayed on the screen 310 of FIG. 16. In this case, based on the biological information and the user behavior information displayed on the screen, the supervisor can determine whether the user recovers his/her health and whether the drug is effective. Hence, when the drug is ineffective or the health recovery is slow, the supervisor can generate the new behavior guideline by modifying the behavior guideline to change the drug or increase the dosage. When determining that the user is completely recovered, the supervisor may delete the behavior guideline for the medication because the user does not need to take the drug any more.

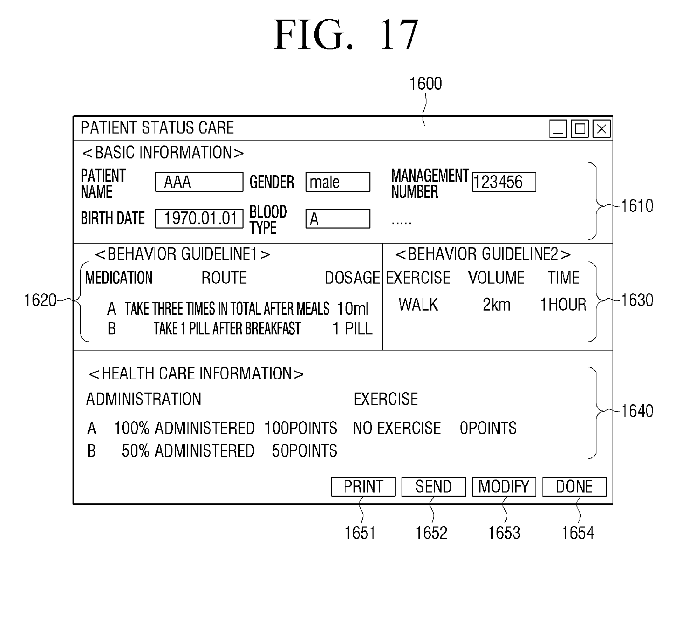

FIG. 17 depicts a screen displaying the health care information. Referring to FIG. 17, the health care information screen 1600 includes a region 1610 for displaying the user's basic information, regions 1620 and 1630 for displaying various behavior guidelines, a region 1640 for displaying the health care information, and various menu items 1651 through 1654 for using the information. The health care information screen 1600 of FIG. 17 can be displayed by the device which generates the health care information. According to various configurations, one of the first device 100, the system server 1000, and the second device 200 can display the screen of FIG. 17.

For example, when the first device 100 displays the screen of FIG. 17, the user can print the health care information screen by selecting the print menu item 1651 and send the health care information to the external device by selecting the send menu item 1652. When the send menu item 1652 is selected, a list of devices for transmitting information to can be displayed. The list displays various information about the system server 1000, the second device 200, the third device 300, and the fourth device 400. The user can select the transmission destination by selecting the information on the list and send the health care information to the destination.

As above, the user's basic information, behavior guideline, and health care information can be displayed by various screens. The screen structure can differ according to the type of the first device 100.

< Various screen structures for the information display >

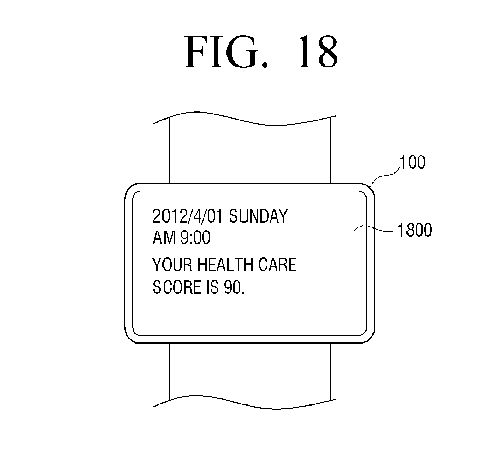

FIGS. 18 through 22 depict various example screens of the first device 100 which manages the user's health using the behavior guideline and the health care information. FIGS. 18 through 22 show the example screens when the first device 100 is wearable on the user's wrist.

Referring to FIG. 18, the first device 100 displays the health care information 1800 converted into an evaluation score. The health care information of FIG. 18 can be displayed continuously or periodically, or only when the user inputs the command. Based on the health care score, the user can be spurred to perform activities to care for their health.

When the evaluation score is accumulated over a preset first threshold, the first device 100 displays a compliment message. When the evaluation score is accumulated below a preset second threshold, the first device 100 displays a warning message. The compliment message or the warning message may be displayed on the first device 100, or provided to the system server 1000 or the second device 200.

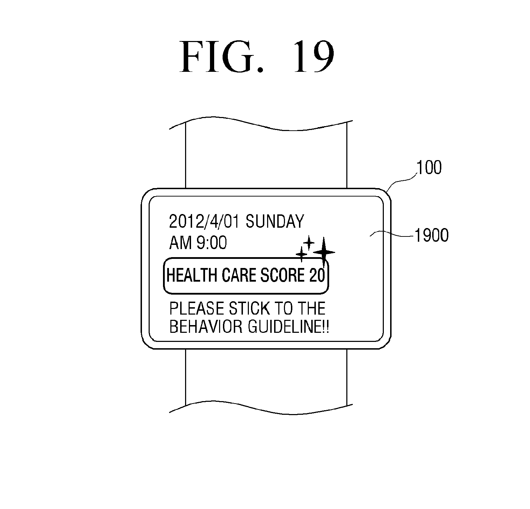

FIG. 19 depicts a screen 1900 displaying the warning message when the evaluation score falls below a preset threshold. In this case, the screen 1900 can be displayed together with the vibration or the notification sound. Alternatively, the color of the screen 1900 displaying the warning message can be changed or flickered to notify of the urgent situation. In this case, a display frequency, a sound volume, a vibration intensity, a light intensity, a notification time, a notification target of the warning message can differ according to the urgency or the evaluation score.

Meanwhile, the above-stated behavior information can be generated based on the information collected through the short-range access of the external object. In this case, upon the access to the external object, a screen inquiring about whether the user actually takes the medicine can be displayed.

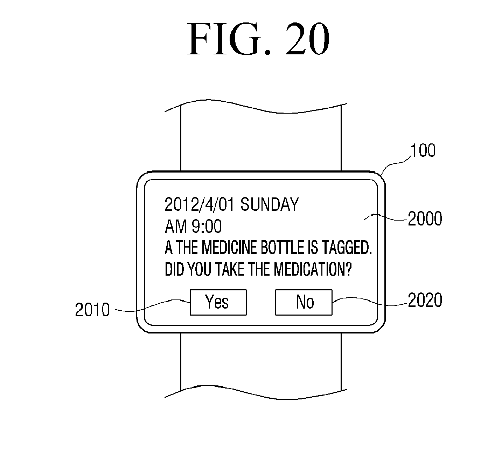

FIG. 20 depicts such a screen. Referring to FIG. 20, when a medicine bottle A is accessed, a screen 2000 including menu items 2010 and 2020 inquiring about the actual drug use is displayed. When the user selects Yes 2010, the behavior information of the corresponding medication is generated. When the user selects No 2020, wrong tagging is determined and the collected data is discarded.

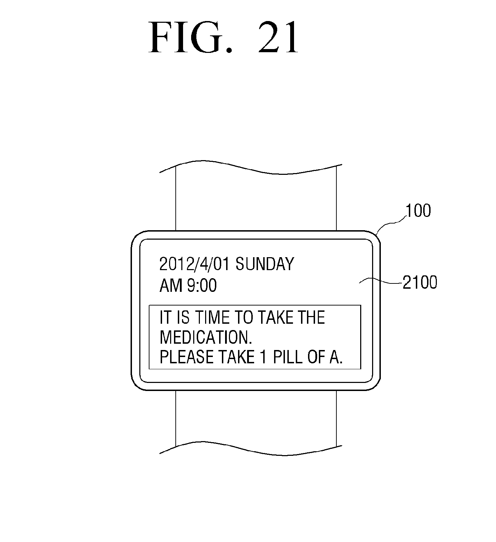

FIG. 21 depicts a screen 2100 for guiding the user to behave according to the behavior guideline. Referring to FIG. 21, the first device 100 displays a screen informing of the medication administration time and dosage. In this case, the vibration or the notification sound can be output together, and the behavior according to the behavior guideline can be induced or encouraged by changing the color of the screen 2100 or switching on and off the screen 2100 repeatedly.

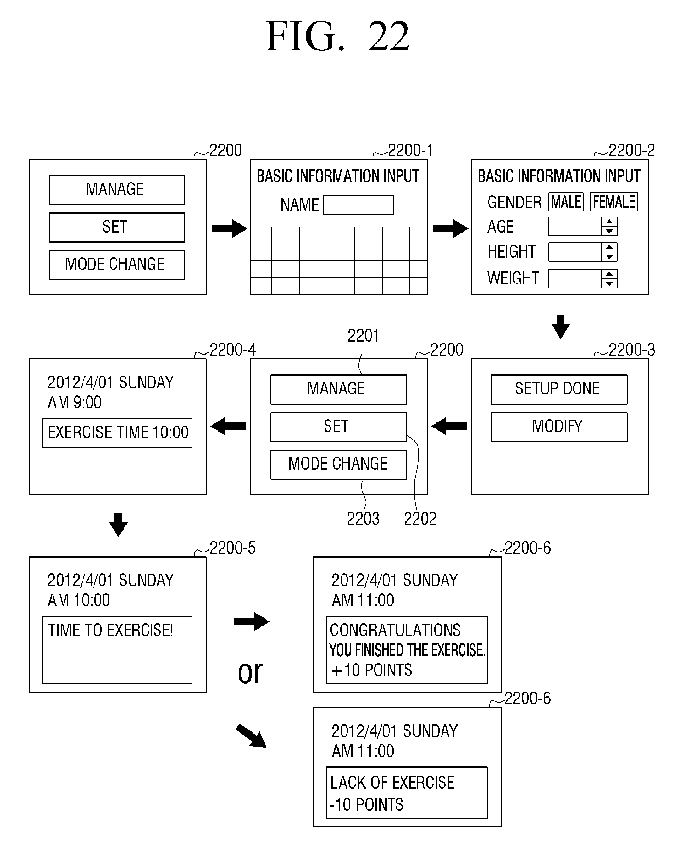

FIG. 22 depicts a sequence of menus and screens showing how to input the user's basic information in the first device 100 and to manage the user's health using the first device.

Referring to FIG. 22, a standby screen 2200 of the first device 100 displays a management menu item 2201 for initiating the health management, a setup menu item 2202 for inputting the basic information, and a menu item 2203 for switching a mode. When the user selects the setup menu item 2202, a screen 2200-1 for inputting the name of the user's basic information is displayed. The screen 2200-1 displays a region for displaying the name and a soft key for entering the name. When the name is input, a screen 2200-2 for inputting the basic information such as gender, age, height, and weight is displayed. When the basic information is input, a screen 2200-3 including a menu for finishing or modifying the setup is displayed. When the setup completion menu is selected in the screen 2200-3, the screen is switched back to the standby screen 2200.

When the user selects the management menu item 2201 on the standby screen 2200, the screen is switched to a management screen 2200-4. The management screen 2200-4 displays information about the activity to do based on the behavior guideline. The management screen 2200-4 of FIG. 22 includes a message notifying or reminding the user of the time to exercise that is an hour away based on the current time of 9:00 AM. When the exercise time arrives, a management screen 2200-5 including a message notifying that it is time to exercise is displayed. The user may begin the exercise according to the message, or disregard the message.

When the user takes the exercise according to the behavior guideline, a management screen 2200-6 displays the positive feedback and the added evaluation score. In contrast, when the user disregards the behavior guideline and does not take the exercise, a management screen 2200-7 displays the negative feedback and the reduced evaluation score.

As above, even when the first device 100 includes the small display screen wearable on the user's wrist, it can easily fulfill the basic information input, the behavior guideline display, the health care information display. The health care information can be displayed using a graph or a drawing instead of the text.

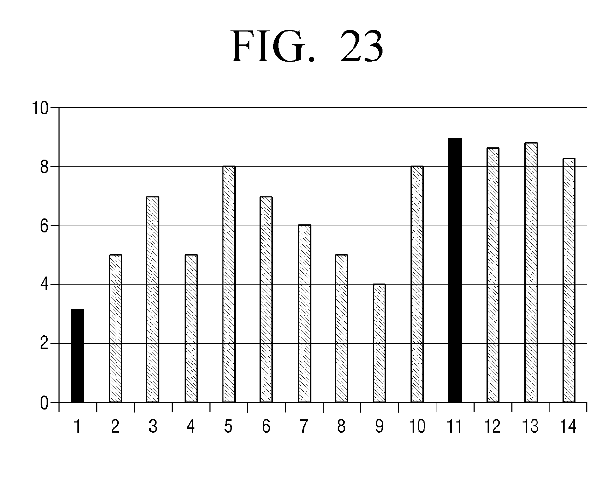

FIG. 23 depicts a graph showing the accumulation of the evaluation score according to the date when the health care information is recorded as the evaluation score. In FIG. 23, the vertical axis indicates the cumulative evaluation score and the horizontal axis indicates the date. On the graph, the date of the best health care or the date of the worst health care can be highlighted by changing its color or flickering. In FIG. 23, the first-day bar of the lowest evaluation score and the eleventh-day bar of the highest evaluation score are colored differently from the other bars.

When the graph of FIG. 23 is displayed, the user can enlarge the graph. When a touch screen function is supported, the user may enlarge the graph by directly touching the graph or by touching the graph with two or more fingers and pinching the touch points apart. When the graph is zoomed in, different graphs showing detailed health care information such as exercise, medication and food can be additionally displayed.

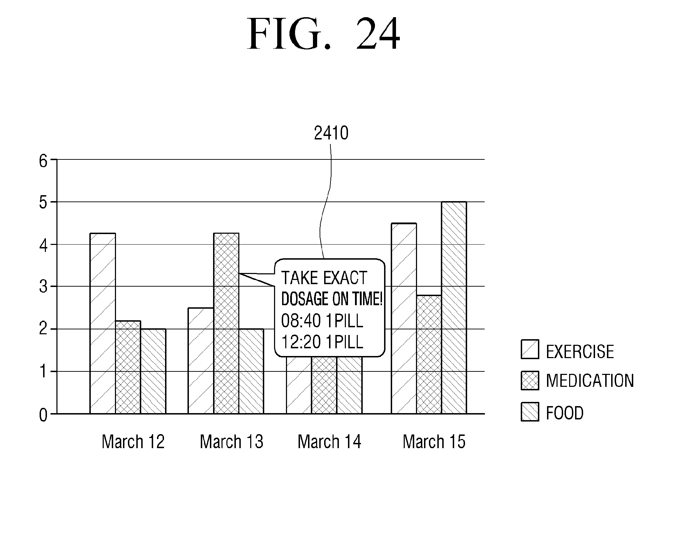

FIG. 24 depicts the zoom-in graph. Referring to FIG. 24, the health care information is generated differently based on the behavior guideline. That is, respective graphs for the exercise guideline, the medication guideline, and the food intake guideline are displayed. The length of the graph indicates the evaluation score. When the user selects the graph in FIG. 24, the screen can additionally display detailed information 2410. In FIG. 24, the detailed information 2410 displays the administration time and the dosage.

While the user enlarges the graph of FIG. 23 to arrive at the graph in FIG. 24, the graph of FIG. 24 may be displayed initially. While a bar graph is depicted in FIGS. 23 and 24, a circle graph or other graphs may be applied and displayed.

The duration for displaying the evaluation score using the graph can be arbitrarily selected by the user. For example, the user can select the unit such as one year, one month, first quarter, and one week, or input the duration in person. Alternatively, each graph can be divided on a weekly basis and displayed in a single page. In this case, the user's touch or scroll may switch to a page showing a previous or following week's evaluation score.

As mentioned above, the user terminal device can be implemented using various devices. When the user terminal device is implemented using a small device wearable on the wrist or attachable to other body part such as belt or ankle, like a pedometer, its display size is limited. The small display device can display the screen in the layout for the display size, and the display information can be simplified.

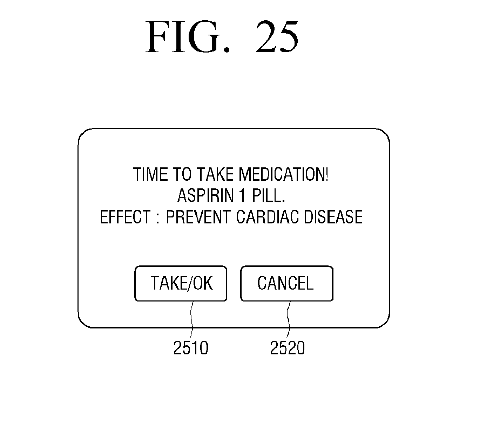

FIGS. 25 through 29 depict various screens displayable by the device including the small display.

Referring to FIG. 25, the screen can include a message notifying the user that it is time to take the drug according to the behavior guideline, the drug name to take, and the dosage, a button 2510 for receiving user input indicating that the drug is taken, and a cancel menu item 2520 which disregards the message. In FIG. 25, although the drug information is not received directly through the short-range wireless communication, when the menu item 2510 for the medication check is selected, the device generates the behavior information about the user's medication administration and accordingly generates the health care information. For example, a certain value can be added to the evaluation score. In this case, the first device 100 can omit the short-range wireless communication reader. In contrast, when the cancel menu item 2520 is selected, the device can generate the behavior information indicating that no medication is taken and accordingly subtract a certain value from the evaluation score.

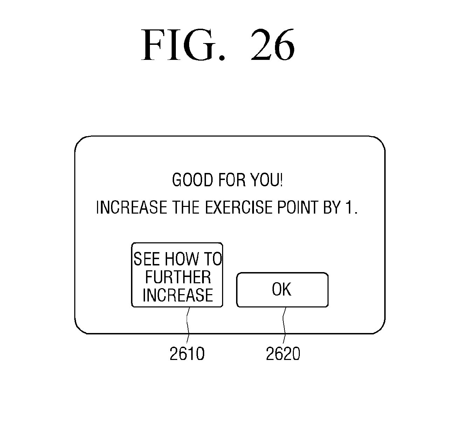

Referring to FIG. 26, the displayed screen can include a congratulatory message when the user's health is cared for successfully and the evaluation score is greater than predetermined value, a menu item 2610 for providing more information on how to increase the evaluation score for exercise may be provided, and an OK menu item 2620 for dismissing the message. When the menu item 2610 is selected in FIG. 26, additional detailed information is displayed about to raise the evaluation score of the user. Hence, user's competitive spirit is stimulated and the user may show more interest in health care.

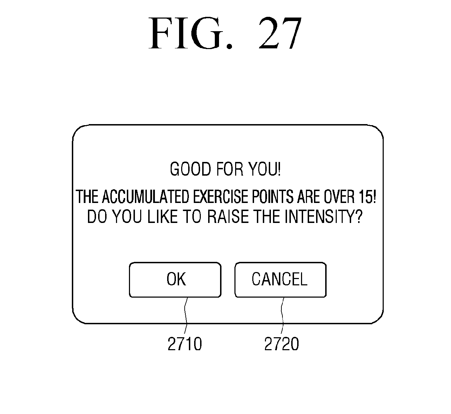

Referring to FIG. 27, when the health of the user is cared for continuously and the cumulative evaluation score exceeds a first threshold, the screen can display a message informing the user of the good health management, a menu item 2710 for raising the behavior guideline, and a cancel menu item 2720 for dismissing the message.

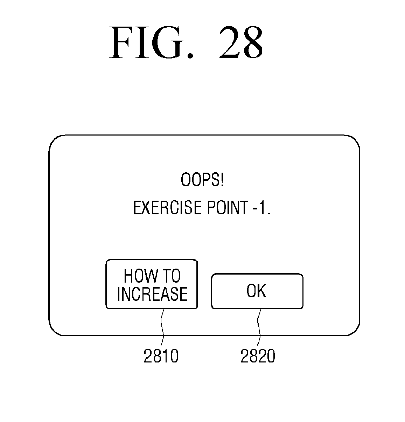

Referring to FIG. 28, when the user's health is managed poorly, the screen can display a message notifying the user of a reduced exercise score, a menu item 2810 for checking additional information to enhance the user's behavior, and an OK menu item 2820 for dismissing the message.

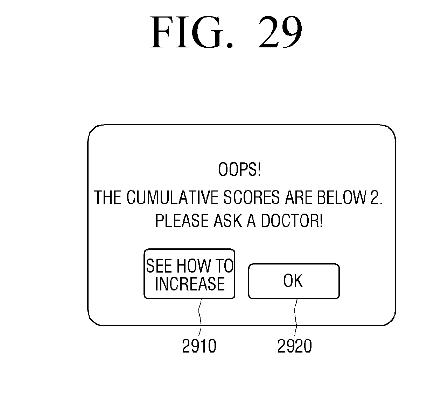

Referring to FIG. 29, when the user's health is health is not cared for continuously and the cumulative score falls below a second threshold, the screen can display a warning message, a menu item 2910 for checking additional information to enhance the user's behavior, and an OK menu item 2920 for dismissing the message. While only two menus items 2910 and 2920 are displayed in FIG. 29, the screen may further include a menu item for sending the evaluation score to an expert or a menu item for requesting a decrease in the behavior guideline. As the screen of FIG. 29 is displayed, the user's health care information may be automatically transmitted to the supervisor at the same time. As discussed above, various information can be provided in various screens when the health management service is provided.

Meanwhile, the first device 100 can be implemented using the user terminal device. The operations of the user terminal device can vary according to implementations. Now, the operations of the user terminal device are elucidated according to implementations.

< Operations in the user terminal device >

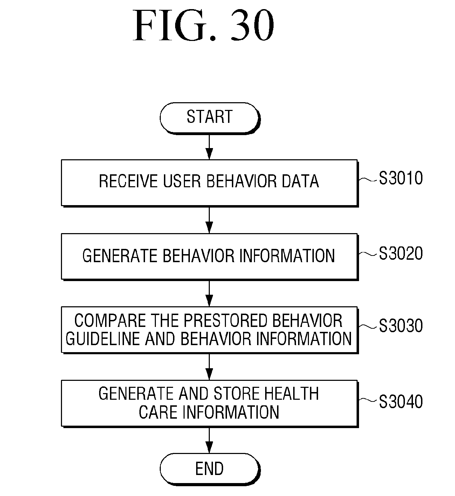

FIG. 30 is a flowchart of a user customized health management method for a user terminal device according to an exemplary embodiment. Referring to FIG. 30, the user terminal device obtains the user's behavior while the behavior guideline is stored, and manages the health of the user by itself.

In detail, the user terminal device receives data about the user's behavior (S3010). The data may be received using the short-range wireless communication, through the direct input, or from the external device as stated earlier.

Using the received data, the user terminal device generates the behavior information (S3020). The behavior information generation has been explained in the exemplary embodiments described above and shall be omitted here.

The user terminal device compares the pre-stored behavior guideline and the behavior information (S3030). The behavior guideline can be manually input by the user to the user terminal device, or automatically generated by the user terminal device. Also, the behavior guideline can be received from the external device. The behavior guideline and the behavior guideline generation can vary according to the implementation, have been described above, and shall be omitted here.

Based on the comparison of the behavior guideline and the behavior information, the user terminal device generates and stores the health care information (S3040). The behavior guideline and the health care information can be used diversely as described in FIGS. 18 through 29.

According to the implementations, the behavior guideline and the health care information can be transmitted to the external device. In particular, when the behavior guideline requires the approval, the approval request is transmitted to the external device. Only when the approval is received, the behavior guideline can be stored and used.

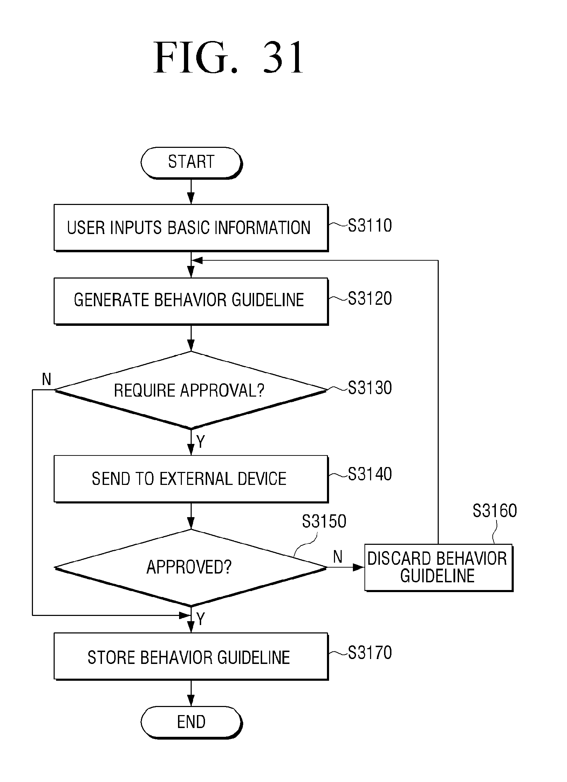

FIG. 31 is a flowchart of a method for creating and storing the behavior guideline in the user terminal device.

Referring to FIG. 31, when the user basic information is input (S3110) and the behavior guideline is generated based on the basic information (S3120), the user terminal device determines whether the behavior guideline requires the approval (S3130).

When the approval is unnecessary, the user terminal device stores the behavior guideline (S3170).

Alternatively, when the approval is necessary, the user terminal device sends the request to the external device (S3140) . Herein, the external device can be the system server 1000 or the second device 200. When the information of the second device 200 is not stored, the user terminal device may gain the approval of the second device 200 through the system server 1000.

Hence, when the approval of the external device is received (S3150), the user terminal device stores the approved behavior guideline (S3170) . Alternatively, when the approval of the external device is not received within a predetermined period of time, the user terminal device discards the generated behavior guideline (S3160) and regenerates the behavior guideline (S3120) .

As such, the user terminal device generates the behavior guideline requiring the external approval and the behavior guideline requiring no external approval to manage the user's health.

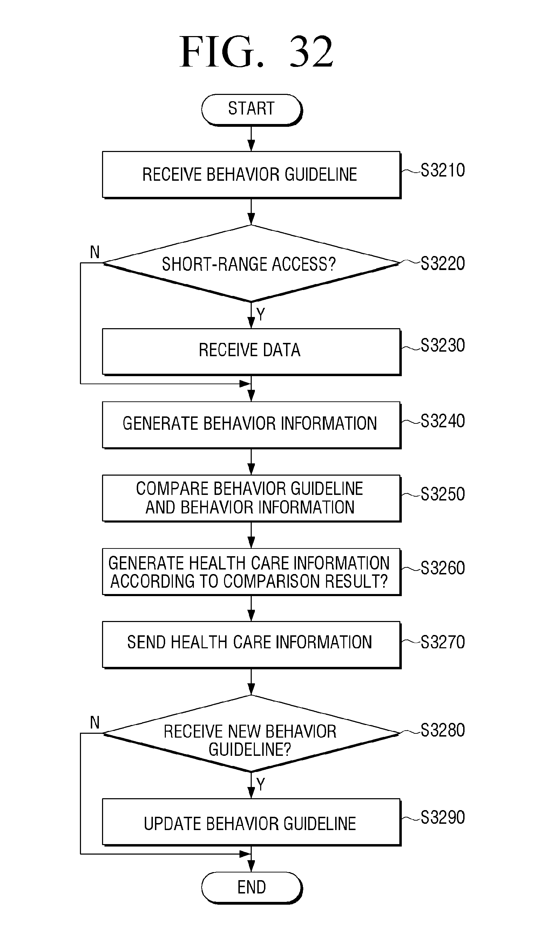

FIG. 32 is a flowchart of a user customized health management method for a user terminal device according to another exemplary embodiment.

Referring to FIG. 32, the user terminal device receives the behavior guideline generated by the external device (S3210). When the user terminal device is implemented using the first device 100 of the system of FIG. 1, the external device can be the second device 200 and the user terminal device can receive the behavior guideline generated by the second device 200 via the system server 1000.

At this time, when nearby objects are accessed by the user terminal (S3220), the user terminal device receives data from the nearby objects (S3230) and generates the behavior information (S3240).

The user terminal device compares the behavior guideline and the behavior information (S3250) and generates the health care information according to the comparison (S3260). The user terminal device sends the generated health care information to the external device (S3270). The health care information may include the feedback information generated based on an evaluation score generated according to the comparison of the behavior guideline and the behavior information, or the text or the graph indicating the match rate or the match level between the behavior guideline and the behavior information. The health care information may include the temporary behavior guideline extracted based on the comparison of the behavior guideline and the behavior information. Additionally, the temporary behavior guideline can be a temporarily modified behavior guideline according to the match level of the behavior guideline and the behavior information. The user terminal device can send the temporary behavior guideline to the external device, gain the approval from the external device, and then replace the previous behavior guideline by the temporary behavior guideline.

When receiving the new behavior guideline from the external device (S3280), the user terminal device updates the behavior guideline with the new behavior guideline (S3290).

The user terminal device then manages the user's health according to the updated behavior guideline. Alternatively, the system server may generate and store the health care information. In this case, the generation and the storing of the health care information in FIGS. 30 through 33 may be omitted.

< Operations in the system server >

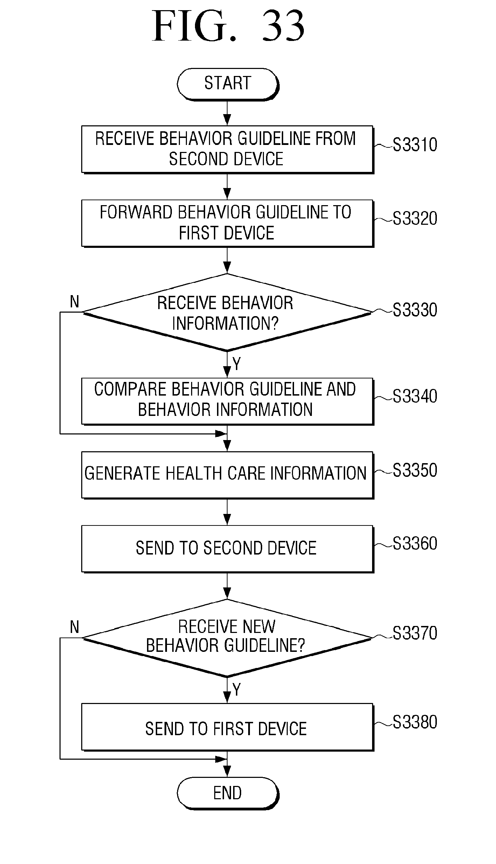

FIG. 33 is a flowchart of a user customized health management method for a system server.

Referring to FIG. 33, the system server 1000 receives the behavior guideline from the second device 200 (S3310). The behavior guideline can be generated based on the user's basic information. The basic information may be input from the first device 100 or the system server 1000 and forwarded to the second device 200, or directly input to the second device 200.

The system server 1000 sends the behavior guideline to the first device 100 (S3320). The system server 1000 stores the behavior guideline in the storage space allocated for the user of the first device 100. After receiving the behavior information from the first device 100 (S3330), the system server 1000 compares the behavior guideline and the behavior information (S3340).

The system server 1000 generates the health care information according to the comparison (S3350). When a preset event occurs, the system server 1000 sends the health care information to the second device 200 (S3360). In this case, the system server 1000 may send the health care information to various external devices such as the first device 100 or the third device 300, besides the second device 200.

When receiving the new behavior guideline from the second device 200 (S3370), the system server 1000 forwards the received new behavior guideline to the first device 100 (S3380).

Hence, the behavior guideline can be adaptively changed according to the user's behavior. For example, for good user health management, the behavior guideline can be intensified to enhance the effect. For poor user health management, the behavior guideline can be loosened to encourage the user to follow the behavior guideline more easily.

Meanwhile, after the user's disease is completely cured or the enough medication is taken, the new behavior guideline may be generated by deleting all or part of the previous behavior guideline. When part of the behavior guideline is deleted, the first device 100 manages the health with the other behavior guidelines excluding the deleted behavior guideline. Alternatively, when all of the behavior guideline is deleted, the first device 100 can manage the health according to the general healthy life habit information. For example, the first device 100 can induce the user to care his/her health by periodically outputting a message encouraging the user to have a meal at a fixed time and to exercise. The healthy life habit information can be transmitted as additional information together with the behavior guideline. The operations of the system server 1000 may differ according to the implementations which have been illustrated.

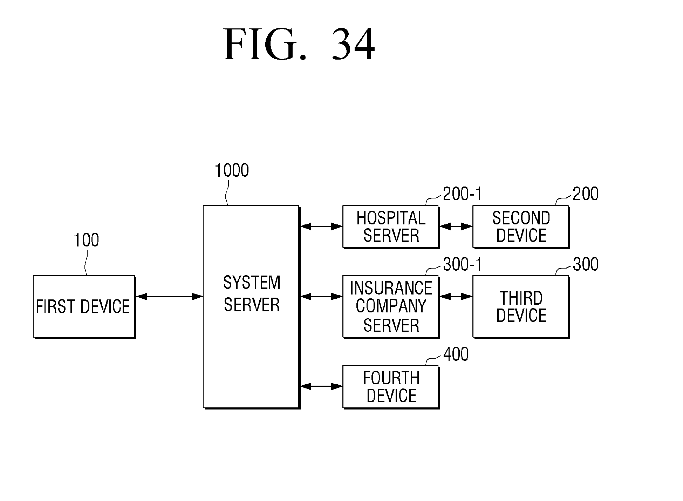

FIG. 34 depicts a health management system for the B2B model. Referring to FIG. 34, the health management system includes the first device 100, the system server 1000, a hospital server 200-1, the second device 200, an insurance company server 300-1, the third device 300, and a guardian terminal device 400.

The user subscribes to the user customized health management service of the administrator of the system server 1000. The service may be subscribed to online through the first device 100 or other user terminal device, or offline. In the service subscription, the user provides the administrator of the system server 1000 with his/her basic personal information such as name, age, resident registration number, gender, weight, and height. In association with other servers using the basic personal information, the administrator of the system server 1000 automatically collects user information. For example, the therapy record, the medical history, the family anamnesis, and the physical constitution information can be collected.

According to the contract with the user, the administrator of the system server 1000 generates the account of the user and allocates the storage space for the account. The administrator sends the user's basic information to the related hospital server 200-1 and insurance company server 300-1.

The hospital server 200-1 selects the supervisor relating to the user's condition among supervisors such as doctors or nurses, and matches information of the second device 200 of the supervisor with the user. Accordingly, when the user's behavior guideline is input to the second device 200, the hospital server 200-1 forwards the behavior guideline to the system server 1000. The system server 1000 sends the behavior guideline to the first device 100 and the fourth device 400 of the guardian.

According to the received behavior guideline, the first device 100 outputs a message for guiding the user's behavior or a message notifying of an activity time according to the behavior guideline, and thus manages the user's health.

The first device 100 generates the behavior information by collecting data about the user's behavior, and sends the generated behavior information to the system server 1000. The system server 1000 stores the received behavior information in the storage space allocated for the user's account, and generates and stores the health care information by comparing the pre-stored behavior guideline and the behavior information. The system server 1000 sends the health care information to the second device 200 via the hospital server 200-1 or directly. Thus, the second device 200 redefines or deletes the behavior guideline by monitoring the user's health care, and systematically manages the user's health.

In addition, the insurance company server 300-1 selects one of insurance brokers assigned and matches the information of the third device 300 of the selected insurance broker with the user. The third device 300 generates insurance data of the user using the insurance fee calculation algorithm and sends the generated insurance data to the system server 1000. In some cases, the third device 300 may send the insurance data to the first device 100 or the fourth device 400.

When the behavior guideline is changed according to the user's health care status, the system server 1000 sends the changed behavior guideline and the health care information back to the insurance company server 300-1. Hence, the third device 300 updates and stores the insurance data based on the received information.

The users of the first device 100 and the fourth device 400 may pay for the user customized health management service using an electronic payment system.

In the system of FIG. 34, the first device 100, the second device 200, the third device 300, and the fourth device 400 can be driven by an application generated to offer the health management service. The administrator, who provides the user customized health management service, can create the application for each device and distribute the application through the system server 1000, a separate web server (not shown), or an application store (not shown).

The user can use the health management service by installing an application on the first device 100.

In the system of FIG. 34, the first device 100 may include, but not limited to, the components of FIG. 2 or FIG. 13 and the system server 1000 may include, but not limited to, the components of FIG. 5. The first device 100 and the system server 1000 may include other different components.

While the hospital server 200-1 and the insurance company server 300-1 are illustrated in FIG. 34, the system can include a health club server (not shown), a rehabilitation clinic server (not shown), a health insurance corporation server (not shown), and a nutritionist terminal device. In this case, the behavior guideline relating to the exercise and the behavior guideline about the rehabilitation program or the diet can be provided to the user by the respective server to manage the overall user's health.

Besides, the system of FIG. 34 can include an emergency rescue center server (not shown) . That is, when the first device 100 includes therein a sensor for detecting various bio- signals such as a user's heart rate, temperature, blood sugar, respiration, blood pressure, motion, and weight, or is connected to the sensor to receive such bio- signals, the user's condition can be continuously monitored using the bio- signals. When determining that the user's condition is critical, the first device 100 can notify the emergency rescue center server of the emergency, the hospital server 200- 1, or the fourth device 400 via the system server 1000 or directly. The emergency notification can also be sent to a network server such as Social Network Service (SNS) server.

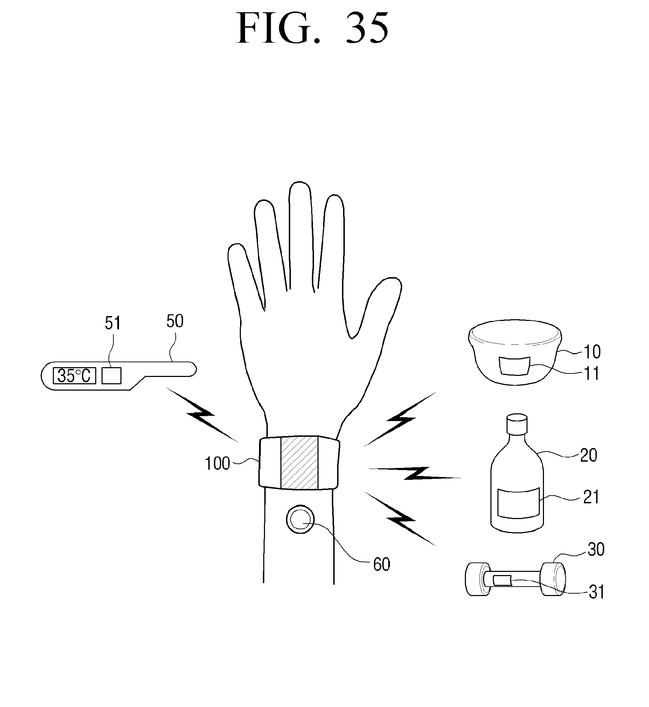

FIG. 35 depicts the first device 100 which obtains the user's condition by collecting data from not only the various external objects 10 through 40 but also various sensors 50 and 60.

Referring to FIG. 35, when a thermometer 50 attached with a short-range wireless communication tag 51 is accessed by the first device 100, a user's temperature information can be collected. The first device 100 may also collect blood sugar information by communicating with the blood sugar sensor 60 attached to the user's body. Additionally, data can be collected from various bio-signals.

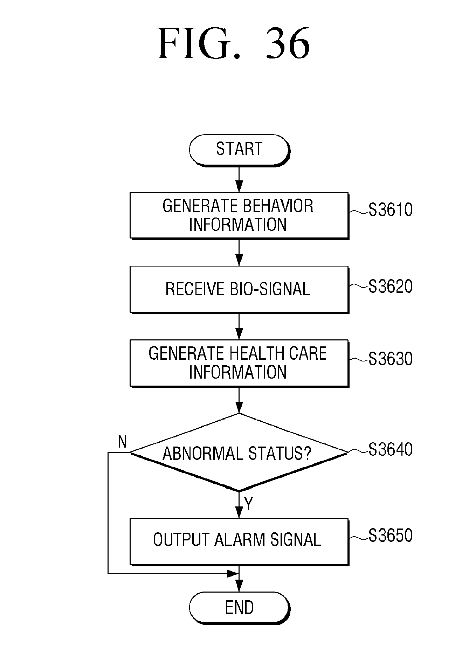

FIG. 36 is a flowchart of a user customized health management method for a user terminal device when the emergency is notified using the bio-signal. Referring to FIG. 36, the user terminal device generates the behavior information by collecting data from various nearby objects (S3610).

The user terminal device receives the bio-signal separately from the behavior information collection (S3620), and generates the health care information by considering all of the bio-signal and the behavior information (S3630).

Hence, the user terminal device can manage the health more accurately than the health care information generation by calculating the evaluation score according to whether the behavior guideline and the behavior information match. That is, the user terminal device determines that the user's health gets better when the behavior information corresponds to the behavior guideline over a certain degree and the bio- signal nears to a reference value. For example, when the behavior guideline is focused on the weight loss, the user consistently sticks to the dietary meal and the exercise as defined by the behavior guideline, and the user's weight gets closer to the goal weight, the user terminal device can determine the user's weight loss is successful. In contrast, when the user's weight is not reduced to the goal weight or changes little although the user follows the behavior guideline, the behavior guideline can be intensified.

Additionally, when the user's bio- signal such as temperature, blood sugar, blood pressure, and heart rate satisfies a preset abnormal condition (S3640), the user terminal device outputs the alarm signal (S3650) . The alarm signal can be output in the form of a visual message, a sound message, and a vibration. The user terminal device may send the alarm signal to an external device, in addition to the alarm signal output by the user terminal. The external device can employ various devices such as system server 1000 and second through n- th devices 200 through n.

While the first device 100 is a user terminal device wearable on the user's wrist in FIGS. 3 and 35, the first device 100 may be implemented using a user terminal device such as mobile phone.

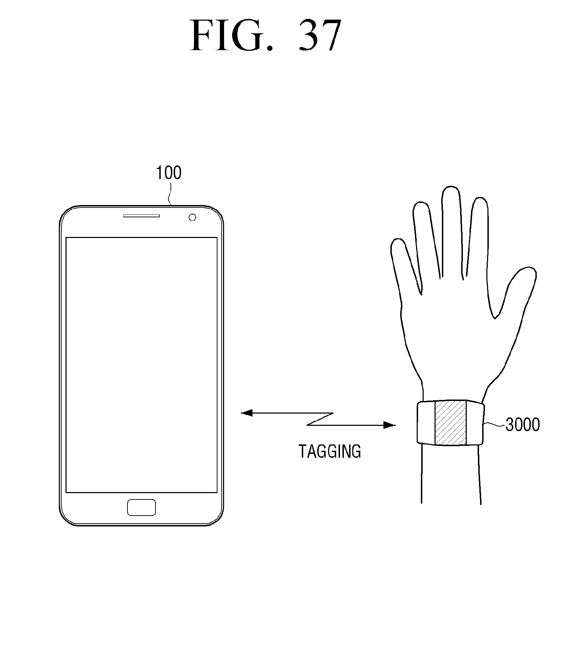

FIG. 37 depicts the first device 100 being the mobile phone.

Referring to FIG. 37, the first device 100 can receive and utilize data generated by a separate information collector 3000. The first device 100 can receive the data using the short-range wireless communication. The short-range wireless communication can include various technologies such as NFC, Bluetooth, Wi-Fi, Zigbee, barcode, and QR code.

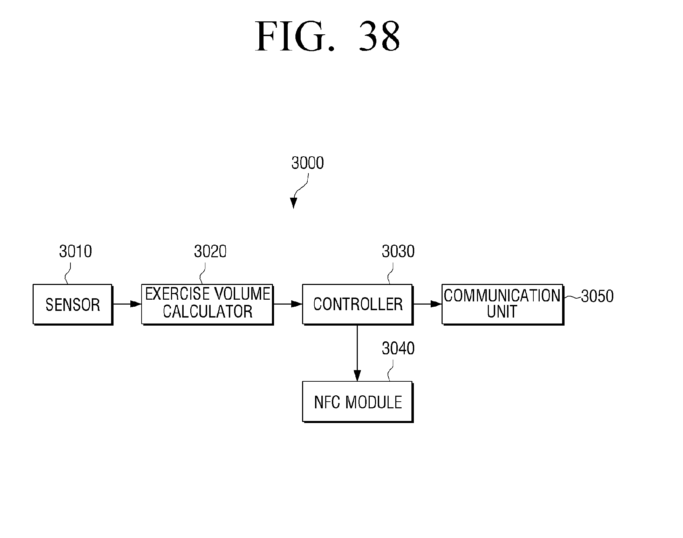

The information collector 3000 can be constructed as shown in FIG. 38.

Referring to FIG. 38, the information collector 3000 includes a sensor 3010, an exercise volume calculator 3020, a controller 3030, a short-range wireless communication module 3040, and a communication unit 3050.

The sensor 3010 senses the user's motion. In detail, the sensor 3010 can include an accelerometer. The accelerometer can include 2 axes or 3 axes. The accelerometer calculates a pitch angle and a roll angle.

The exercise volume calculator 3020 analyses the user's exercise volume by using the value sensed by the sensor 3010. For example, the exercise volume calculator 3020 calculates the exercise time, a pace count, a movement distance, a calorie consumption, a total calorie consumption, an exercise intensity, and a basal metabolic rate. Among them, information such as calorie consumption, calorie consumption per mile, movement distance, and calorie consumption during jogging can be calculated using the following equations.

[Equation 1]

$$CC = MC*M*0.00006213$$

$$MC = 3.7103 + (0.2678*W) + (0.0359*(P*60*0.00006213)*2)*W$$

$$M = ((H=100)*P)/100$$

$$JC = (33.3 + 0.178*(JP-150)*W)/100$$

M denotes the movement distance, MC denotes the calorie per mile, CC denotes the calorie consumption, W denotes the weight, P denotes the pace count, H denotes the height, JC denotes the calorie consumption during jogging, and JP denotes the jogging pace count.

When the exercise volume is calculated according to the various items as expressed in Equation 1, the behavior guideline can be set according to various items such as movement distance, calorie consumption, calorie per mile, and jogging pace count.

For example, when the user terminal device itself generates the behavior guideline and the user inputs the current weight, the goal weight, and the exercise period in the basic information, the weight to reduce within the exercise period is calculated. Hence, a maximum intake calorie a day and a calorie to consume through the daily exercise are calculated, and the jogging pace count and distance corresponding to the calorie consumption calculated based on Equation 1 can be calculated. Therefore, an adequate behavior guideline for the user can be established.

The communication unit 3050 receives the bio-signals by communicating with the nearby sensors. For example, the communication unit 3050 can receive the bio-signals from a scale, a blood glucose monitor, an ictometer, and a blood pressure manometer. The communication unit 3050 can communicate with the nearby sensors using a wired communication technique besides the wireless communication techniques such as Wi-Fi, Bluetooth, and Zigbee. While the communication unit 3050 is separated from the NFC module 3040 in FIG. 38, the NFC module 3040 can be used to receive the information of the nearby sensors when the nearby sensors include an NFC tag. In this case, the communication unit 3050 can be omitted.

The controller 3030 stores the exercise volume calculated by the exercise volume calculator 3020 and the bio-signals received at the communication unit 3050, in the NFC module 3040. The NFC module 3040 includes the NFC tag. In some cases, the NFC module 3040 can include an NFC reader together with the NFC tag.

The NFC tag can include an Integrated Circuit (IC) and an antenna coil. When the first device 100 including the NFC reader is tagged, the NFC module 3040 is driven by radio waves emitted by the NFC reader and sends an RF signal carrying information stored in the NFC tag. The antenna coil in the NFC tag induces currents according to the radio waves emitted by the NFC reader. The induced currents are charged to a capacitor of the NFC tag. The IC is driven by the currents charged in the capacitor to generate the RF signal by modulating and coding the pre-stored information.

The NFC module 3040 can generate the RF signal according to preset modulation and coding schemes of various modulation and coding schemes. That is, the modulation loads data by shifting an amplitude, a frequency, and a phase of RF carrier signals exchanged between the NFC tag and the reader. The modulation can use Amplitude Shift Keying (ASK), Frequency Shift Keying (FSK), and Phase Shift Keying (PSK). The ASK shifts the amplitude of the carrier signal according to whether a digital information signal is 0 or 1. For example, the amplitude of the carrier signal is reduced when the information signal is 0 and increased when the information signal is 1. 2-level amplitude can carry 1 bit, and 4-level different amplitudes can carry 2 bits concurrently. The FSK allocates 0 and 1 bits being the digital signal to two-type frequencies (low frequency and high frequency). For example, the information signal of 0 is transmitted over a lower frequency than the carrier frequency, and the information signal of 1 is transmitted over a higher frequency than the carrier frequency. The PSK shifts the phase of the carrier wave according to the data to transmit. The shift of the phase is determined by the data. The information is transmitted bit by bit by shifting the phase of the carrier wave by 180 degrees when the data to transmit is 0 and by 90 degrees when the data is 1. The coding scheme can use a Modified Miller coding and a Manchester coding.

The modulation and coding schemes can be properly selected according to the type and the environment of the device. For example, when the NFC tag is a passive type which is unpowered and driven by the current induced by the radio waves from the NFC reader, the ASK and the Manchester coding can be applied. By contrast, when the NFC tag is an active type which includes its own power supply and communicates with the external NFC reader, the ASK and the Modified Miller coding can be applied at the rate of 106 kbps and the ASK and the Manchester coding can be applied at the rate of 212 kbps and 424 kbps.

As above, various data required to generate the user's behavior information can be collected and stored by the information collector 3000, and then transmitted to the first device 100 when accessing the first device 100.

The first device 100 can generate the behavior information using the data received from the information collector 3000, and provide the health management service using the behavior information and the behavior guideline.

Meanwhile, the first device 100 may include various sensors therein to thus directly sense the data. The system server 1000 may be implemented using a micro- server and embedded in the first device 100.

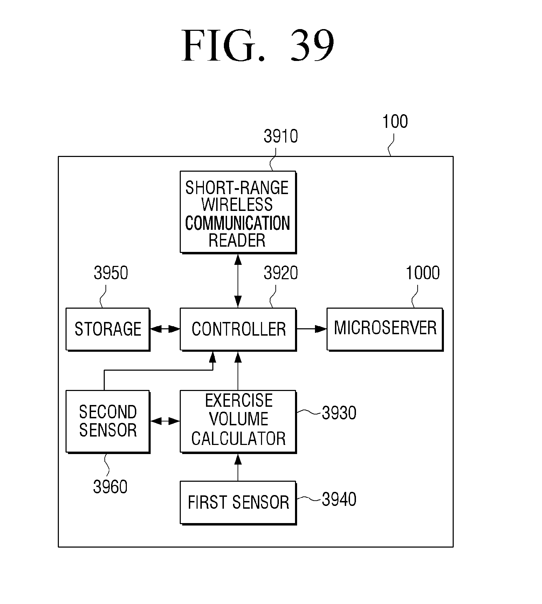

FIG. 39 is a block diagram of the first device 100 when it includes various sensors and the system server 1000.

Referring to FIG. 39, the first device 100 includes a short-range wireless communication reader 3910, a controller 3920, an exercise volume calculator 3930, a first sensor 3940, a storage 3950, a second sensor 3960, and a server 1000.

The first sensor 3940 includes a sensor for sensing the user's motion. The exercise volume calculator 3930 calculates the exercise volume of the user by analyzing the motion sensed by the first sensor 3940. The controller 3920 stores the calculated exercise volume to the storage 3950.

The second sensor 3960 senses the biological information such as user's temperature, blood sugar, and blood pressure. The biological information sensed by the second sensor 3960 can differ according to the position of the second sensor 3960 or the first device 100. For example, when the position and the type (e.g., a wrist band type) of the second sensor 3960 or the first device 100 can measure the blood pressure, the second sensor 3960 can sense the blood pressure. When the position and the type (e.g., a hair band type) of the second sensor 3960 or the first device 100 are attached to the skin, the second sensor 3960 can sense the temperature.

The controller 3920 stores the data read by the short-range wireless communication reader 3910, the exercise volume calculated by the exercise volume calculator 3930, and the biological information sensed by the second sensor 3960 to the storage 3950. Using the stored information, the controller 3920 can generate the behavior information and the health management information. The generated information can be provided to the server 1000. The server 1000 can perform the operations of the system server as described in the various embodiments.

While the system server 1000 is implemented as the micro-server and embedded in the first device 100 in FIG. 39, the system server 1000 may be implemented using a micro-server embedded in the device such as second device 200, third device 300, and fourth device 400.

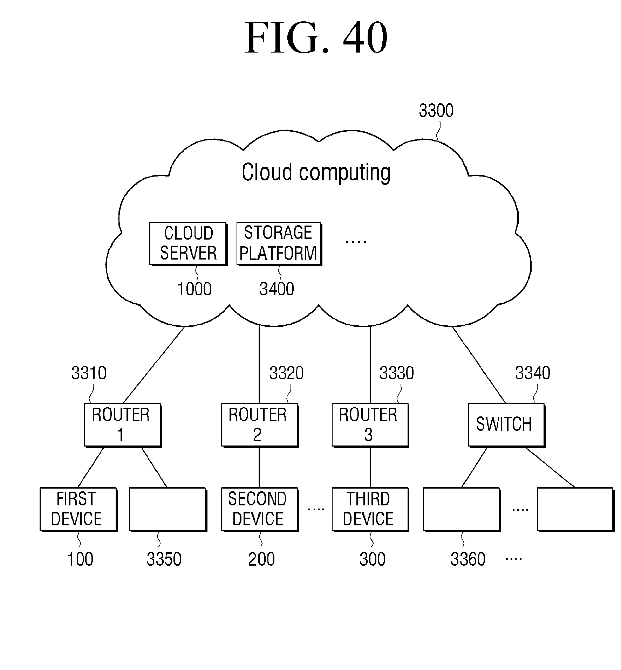

FIG. 40 depicts the system of FIG. 1, which is a cloud network system 3300. That is, the system server can be implemented using a cloud server 1000. The cloud network system 3000 can include the cloud server 1000 and various platforms 3400.

The first device 100, the second device 200, the third device 300, and other devices 3350 and 3360 can be connected to the cloud network system 3000 via a network relay devices such as routers 3310, 3320, and 3330 and switch 3340. Hence, the user customized health management service can be provided according to various implementations.

Meanwhile, the first device 100 can be implemented using various devices such as mobile phone, PDA, tablet PC, MP3 player, laptop computer, and electronic notebook. According to characteristics of the device, various components can be added to the first device 100.

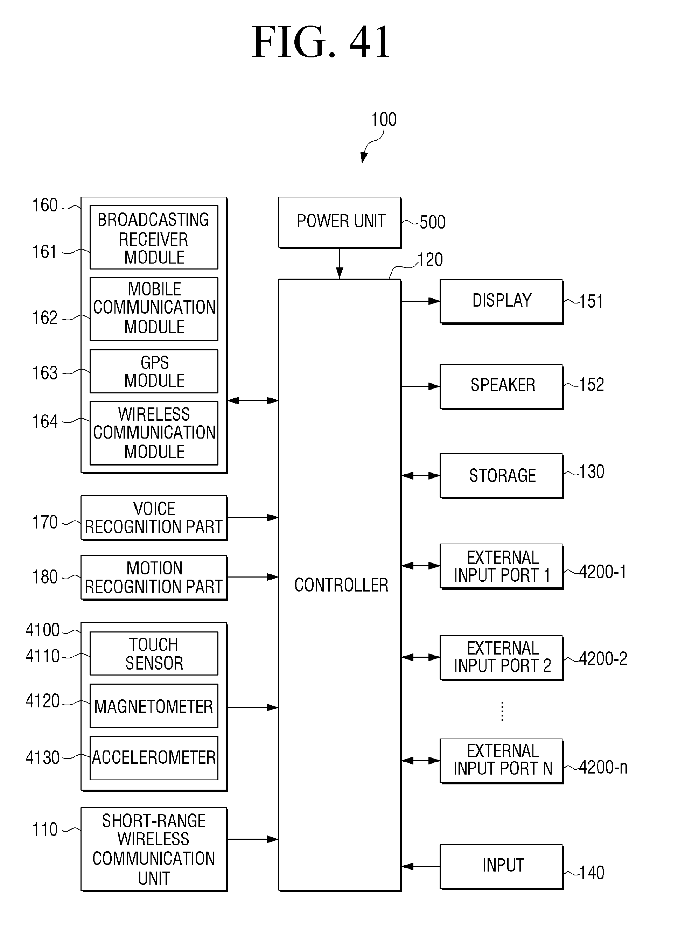

FIG. 41 is a block diagram of various components which can be added to the first device 100.

Referring to FIG. 41, the first device 100 includes a short-range wireless communication unit 110, a controller 120, a storage 130, an input 140, a display 151, a speaker 152, a communication unit 160, a voice recognition part 170, a motion recognition part 180, a sensor 4100, external input ports 4200-1 through 4200-n, and a power unit 500.

The display 151 includes a display panel, a backlight unit, and so on. The display 151 displays various information input screens or information display screens.

The storage 130 can store various programs or data relating to the operations of the first device 100, user's setup information, system operating software, various application programs, and operation information corresponding to the user's control.

The sensor 4100 detects the motion or the status of the user wearing the first device 100. In FIG. 41, the sensor 4100 can include various sensors including a touch sensor 4110, a magnetometer 4120, and an accelerometer 4130. In addition, the sensor 4100 may further include various sensors such as temperature sensor, heart beat sensor, and blood sugar sensor.

The touch sensor 4110 can adopt a capacitive type or a resistive type. The touch sensor 4110 is embedded in the display 110. When the user touches a surface of the display 110, the touch sensor 4110 detects and notifies the touch to the controller 120. The controller 120 can calculate coordinates of the touch point and determine which menu the user selects on the screen.

The magnetometer 4120 detects rotation and movement direction of the first device 100, and the accelerometer 4130 detects acceleration and the tilt of the first device 100. The controller 120 can detect the user's motion using output values sensed by the magnetometer 4120 and the accelerometer 4130, to thus determine the user's exercise volume, intensity, and time. The determined information can be applied to the behavior information.

The communication unit 160 communicates with various external devices using various communication schemes. The controller 120 can send and receive the behavior information, the behavior guideline, the health care information, and other messages to and from the outside through the communication unit 160.

The communication unit 160 can include various communication modules of a broadcasting receiver module 161, a mobile communication module 162, a satellite positioning module (e.g. GPS module) 163, and a wireless communication module 164.

Herein, the broadcasting receiver module 161 can include a terrestrial broadcasting receiver module (not shown) including an antenna for receiving a terrestrial broadcasting signal, a demodulator, and an equalizer, and a DMB module for receiving and processing a DMB broadcasting signal. When the first device 100 is a mobile device such as mobile phone with the broadcasting receiving function, the broadcasting receiver module 161 is required.

The mobile communication module 162 accesses a mobile communication network for the communication in conformity with various mobile communication standards such as 3rd Generation (3G), 3rd Generation Partnership Project (3GPP), and Long Term Evolution (LTE).

The GPS module 163 receives a GPS signal from a GPS satellite and detects the current location of the first device 100. Using the GPS module 163, the controller 120 can calculate the user's movement distance and speed by detecting the location of the user at different points in time. Hence, the controller 120 can calculate the user's exercise volume.

The wireless communication module 164 accesses an external network for the communication in conformity with a wireless communication protocol such as Wi-Fi and/or any other suitable IEEE based protocol.

By use of the mobile communication module 162 or the wireless communication module 164, the controller 120 can communicate with the system server 1000 and other devices.

The short-range wireless communication unit 110 includes the short-range wireless communication reader or the short-range wireless communication tag as mentioned above, and communicates with the external device using the short-range wireless communication. The controller 120 can collect various data relating to the user's behavior from the external devices through the short-range wireless communication unit 110. The short-range wireless communication unit 110 can perform the short-range wireless communication in conformity to the communication protocol such as NFC, Wi-Fi, Zigbee, and Bluetooth.

According to various user selection signals input through the input 140 and the touch sensor 4110, the controller 120 can conduct various operations by selectively activating the components. In detail, the controller 120 can provide the health management service according to various implementations. Besides, the controller 120 may recognize voice input or motion input in addition to the touch manipulation, and perform the operation corresponding to the input. In this case, the controller 120 can activate the voice recognition part 170 and the motion recognition part 180.

The voice recognition part 170 collects user's voice or external sound using a voice acquisition means such as microphone (not shown) and sends it to the controller 120. In a voice control mode, when the user's voice matches a preset voice command, the controller 120 can perform a task corresponding to the user's voice.

The motion recognition part 180 captures a user's image using an image capturing means (not shown) such as camera, and provides the image to the controller 120. In a motion control mode, the controller 120 analyzes the user's image and performs the operation corresponding to the motion gesture when determining the user's motion gesture corresponding to a preset motion command.

The controller 120 provides the health management service according to the user's voice command and motion command input through the voice recognition part 170 and the motion recognition part 180. Specifically, the controller 120 can perform the short-range wireless communication with the external object or the communication with the external devices. Besides the aforementioned health management service, the controller 120 may offer various services such as broadcasting reception service and content play service. These services can be provided by executing the application stored in the storage 130.

The external input ports 4200-1 through 4200-n can be connected to a variety of external devices respectively and receive data, programs and control commands. In detail, the external input ports 4200-1 through 4200-n can include a USB port, a headset port, and so on. In addition to the short-range wireless communication, the controller 120 may receive data from the external object through at least one of the external input ports 4200-1 through 4200-n.

The power unit 500 supplies power to the components of the first device 100. The power unit 500 can include a positive current collector, a positive electrode, electrolyte, a negative electrode, a negative current collector, and a coating part covering them. The power unit 500 may be implemented using a rechargeable secondary battery. The power unit 500 can be implemented using a rechargeable secondary battery such as lithium-ion battery.

While various components of the first device 100 are depicted in FIG. 41, it is not necessary that the first device 100 include all of the components. That is, some of the components can be omitted or added according to the product type of the first device 100, and or replaced by other components.

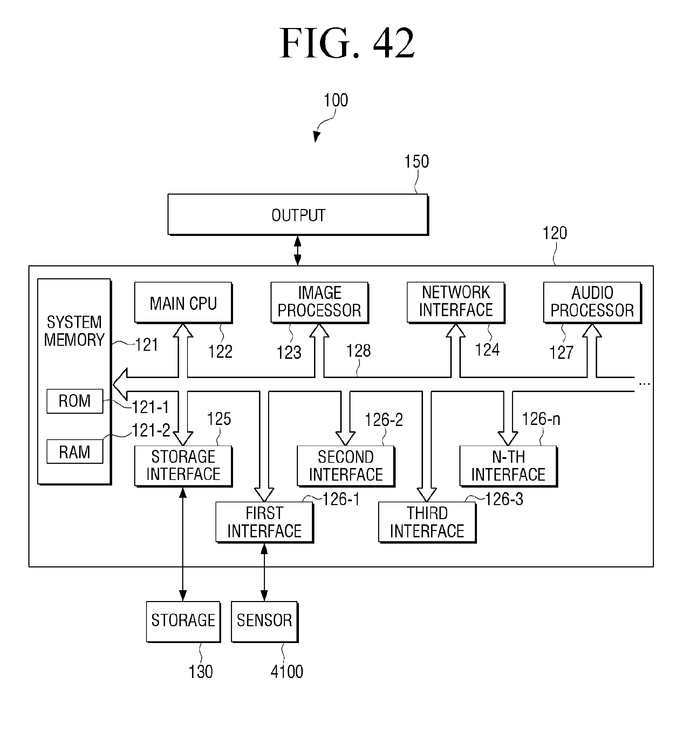

FIG. 42 is a detailed block diagram of the controller 120.

Referring to FIG. 42, the controller 120 includes a system memory 121, a main CPU 122, an image processor 123, a network interface 124, a storage interface 125, first through n-th interfaces 126-1 through 126-n, an audio processor 127, and a system bus 128.

The system memory 121, the main CPU 122, the image processor 123, the network interface 124, the storage interface 125, the first through n-th interfaces 126-1 through 126-n, and the audio processor 127 are interconnected through the system bus 128 to transmit and receive various data and signals.

The first through n-th interfaces 126-1 through 126-n support interfacing between the various components including the sensor 4100 and the components of the controller 120.

The sensor 4100 of FIG. 42 is connected only through the first interface 126-1. When the sensor 4100 includes the various sensors as shown in FIG. 41, each of the sensors can be connected through the interface. At least one of the first through n-th interfaces 126-1 through 126-n may be implemented using a button of a main body of the first device 100, or an input interface for receiving various signals from the external device connected via first through n-th external input ports.

The system memory 121 includes a read only memory (ROM) 121-1 and a random access memory (RAM) 121-2. The ROM 121-1 stores an instruction set for the system boot-up. When a turn-on instruction is input and the power is supplied, the main CPU 122 copies O/S stored in the storage 130 to the RAM 121-2 and boots up the system by executing the O/S according to the instruction stored to the ROM 121-1. When the booting is completed, the main CPU 122 copies various application programs stored in the storage 130 to the RAM 121-2, and performs various operations by running the application programs copied to the RAM 121-2.

By running the application program stored in the storage 130, the main CPU 122 can receive data from the external object and generate the behavior information by processing the data. The main CPU 122 can generate the health care information by comparing the behavior guideline and the behavior information according to the application program execution. Alternatively, the main CPU 122 may provide the system server 1000 with various information such as user's basic information, behavior information, and health care information, and thus offer the health management service together with the system server 1000.

The storage interface 125 is connected to the storage 130 to transmit and receive various programs, contents, and data.

The image processor 123 can include a decoder, a renderer, a scaler, and so on. The image processor 123 decodes the data received from the system server 1000 or the external devices, constructs a frame by rendering the decoded data, and scales a size of the constructed frame in accordance with the screen size of the display 151. The image processor 123 provides the processed frame to the display 151 to display the frame. The generated frame can include various screen frames of FIGS. 14 through 29.

The audio processor 127 processes and provides audio data to a sound output means such as speaker 152. The audio processor 127 reads various alarm sound data from the storage 130 and generates the alarm sound signal using the read data. The generated alarm sound signal can be output through the speaker 152. Also, various information such as basic information, behavior information, and health care information may be generated as voice signals and output through the speaker 152.

The network interface 124 is connected to the external devices over the network. For example, when a web browser program is executed, the main CPU 122 accesses the web server through the network interface 124. When receiving web page data from the web server, the main CPU 122 generates a web page screen by controlling the image processor 123 and displays the generated web page screen in the display 151. When the system server 1000 is the web server, the controller 120 can access the system server 1000 through the network interface 124.

The above-stated various operations of the controller 120 can be executed by running various programs stored to the storage 130.

Various components of the controller 120 according to an exemplary embodiment are depicted in FIG. 42. The skilled person will appreciate that, according to various alternative implementations, some or all of the components can be omitted, changed, substituted, modified, and/or one or more components may be added.

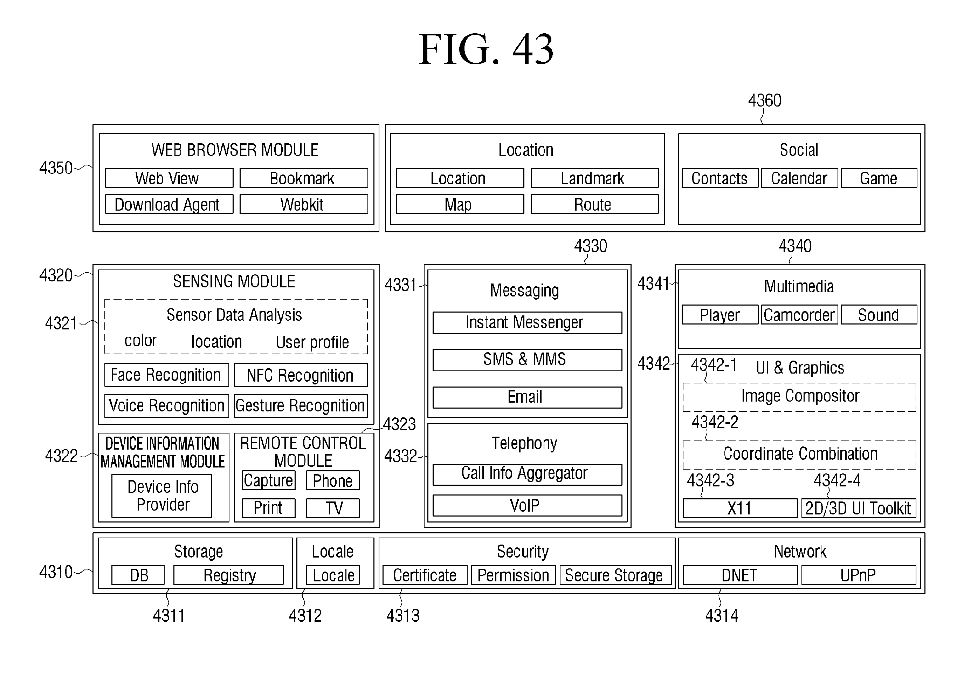

FIG. 43 depicts software architecture of the storage 130 to support the operations of the controller 120 according to various aspects of exemplary embodiments. Referring to FIG. 43, the storage 130 includes a base module 4310, a device management module 4320, a communication module 4330, a presentation module 4340, a web browser module 4350, and a service module 4360.

The base module 4310 is a base module for processing signals received from the hardware of the first device 100 and sending the processed signals to a higher layer module.

The base module 4310 includes a storage module 4311, a location based module 4312, a security module 4313, and a network module 4314.

The storage module 4311 is a program module for managing a database (DB) or a registry. The location based module 4312 is a program module for supporting a location based service in association with hardware such as GPS chip. The security module 4313 is a program module for supporting hardware certification, request permission, and secure storage. The network module 4314 supports the network connection and includes a DNET module and an UPnP module.

The device management module 4320 manages and utilizes the external input and the external device information. The device management module 4320 can include a sensing module 4321, a device information management module 4322, and a remote control module 4323.

The sensing module 4321 analyzes sensor data provided from the various sensors of the sensor 4100. The sensing module 4321 can include a face recognition module, a voice recognition module, a motion recognition module, and an NFC recognition module. The device information management module 4322 provides information about various devices, and the remote control module 4323 is a program module for remotely controlling peripheral devices such as phone, TV, printer, camera, and air conditioner.

The communication module 4330 is a module for communicating with the outside. The communication module 4330 can include a messaging module 4331 such as messenger program, Short Message Service (SMS) and Multimedia Message Service (MMS) program, and Email program, and a telephony module 4332 including a Call Info Aggregator program module and a VoIP module.

The presentation module 4340 is a module for generating the display screen. The presentation module 4340 includes a multimedia module 4341 for playing and outputting multimedia content, and a UI & graphics module 4342 for processing the UI and graphics. The multimedia module 4341 can include a player module, a camcorder module, and a sound processing module. Accordingly, the multimedia module 4341 plays various multimedia contents, and generates and plays the screen and the sound. The UI & graphics module 4342 can include an image compositor module 4342-1 for combining images, a coordinate combination module 4342-2 for combining and generating coordinates on the screen for displaying the image, an X11 module 4342-3 for receiving events from the hardware, and a 2D/3D UI toolkit 4342-4 for providing a tool for creating the 2D or 3D UI.

The web browser module 4350 accesses the web server through the web browsing. The web browser module 4350 can include various modules such as web view module for creating the web page, download agent module for downloading, bookmark module, and Webkit module.

The service module 4360 indicates an application module for providing various services. For example, the service module 4360 can include various modules such as navigation service module for providing map, current location, landmark, and route information, game module, and advertisement application module. In detail, the service module 4360 can include the application for executing the health management service according to various implementations.

The main CPU 122 of the controller 120 accesses the storage 130 through the storage interface 125, duplicates the various modules stored in the storage 130 to the RAM 121-2, and works according to the operation of the duplicated module.

In detail, using the sensing module 4321, the main CPU 122 can analyze the output values of the sensors of the sensor 4100 and generate the behavior information by obtaining the user's status. The main CPU 122 checks the registry of the storage module 4311, detects the pre-stored behavior guideline of the corresponding user, and compares the behavior guideline and the behavior information. The main CPU 122 can generate the health care information according to the comparison and store it in the storage 130. The main CPU 122 can determine whether it is the time to operate according to the behavior guideline, and control the image processor 123 and the audio processor 127 to output the corresponding notification message or sound.

The main CPU 122 may send various information such as a user's basic information, behavior information, behavior guideline, and health care information to the external device such as system server 1000 through the communication unit 160.

As above, the storage 130 can store programs of diverse structures, and the controller 120 can offer the health management service using the various programs stored in the storage 130 according to various exemplary embodiments.

While the software architecture applicable to the first device 100 is illustrated in FIG. 43, the software of this type can be applied to the system server 1000 to offer the health management service.

As set forth above, the health management system can easily collect the user's various information, determine the behavior guideline appropriate for the user, and manage the user's health based on the behavior guideline. While various exemplary embodiments of the present invention are illustrated and described herein individually to ease understanding, the skilled person will appreciate that these embodiments can be realized alone or in any suitable combination.

To use the user customized health management service, the application for the service can be installed in one or more devices.

Specifically, an application for receiving the data about the user's behavior, generating the user's behavior information using the received data, comparing the pre- stored behavior guideline and the behavior information, and generating and storing the user's health care information based on the comparison can be installed in the user terminal device.

In the system server, an application for receiving the behavior guideline of the first device user from the second device, forwarding the behavior guideline to the first device, receiving the behavior information from the first device, generating the health care information by comparing the behavior information and the behavior guideline, forwarding the health care information to the second device, receiving the new behavior guideline from the second device, and forwarding the new behavior guideline to the first device can be installed.

In the second device, an application for displaying the behavior guideline input screen based on the user basic information, sending the behavior guideline input on the behavior guideline input screen to the system server, and generating and sending the new behavior guideline to the system server when the user's behavior information and health care information are received can be installed.

In the third device, an application for generating the insurance data of the user based on the basic information and the behavior guideline, and updating the insurance data when receiving the user's behavior information, new behavior guideline, and health care information can be installed.

Those applications can, for example, be downloaded from the web server, the system server, or various sources, or stored in a non-transitory computer readable medium and provided to the server and the devices.

The non-transitory computer readable medium indicates a medium for storing data semi-permanently and allowing for the reading of the medium by a device, rather than a medium for storing data for a short term such as register, cache, and memory. More specifically, the various application and programs as stated above can be stored to and provided by the non-transitory computer readable medium such as a CD, a DVD, a hard disc, a Blu-ray disc, a USB device, a memory card, and a ROM.

It will be appreciated that embodiments of the present invention can be realized in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape or the like.

It will be appreciated that the storage devices and storage media are embodiments of machine-readable storage that are suitable for storing a program or programs comprising instructions that, when executed, implement embodiments of the present invention. Accordingly, embodiments provide a program comprising code for implementing apparatus or a method as claimed in any one of the claims of this specification and a machine-readable storage storing such a program. Still further, such programs may be conveyed electronically via any medium such as a communication signal carried over a wired or wireless connection and embodiments suitably encompass the same.

Although a few exemplary embodiments have been shown and described, it will be appreciated by those skilled in the art that various changes and modification may be made in these exemplary embodiments without departing from scope of the invention, as defined by the appended claims.

## Claims

1.  A user customized health management method for a user terminal device, the method comprising the steps of:

    receiving data about a behavior of a user;
    generating behavior information of the user using the received data;
    comparing a pre-stored behavior guideline and the behavior information; and
    generating and storing health care information according to the comparison.

2.  The user customized health management method of claim 1, further comprising:

    inputting information (e.g. basic information) of the user; and
    generating and storing the behavior guideline based on the information of the user.

3.  The user customized health management method of claim 1 or 2, further comprising:

    receiving the behavior guideline from an external device; and
    storing the behavior guideline.

4.  The user customized health management method of claim 1, 2 or 3, wherein the health care information comprises feedback information which is generated according to an evaluation score generated based on the comparison of the behavior guideline and the behavior information.

5.  The user customized health management method of claim 4, further comprising:

    outputting a feedback signal according to the feedback information when a preset event occurs,
    wherein the event comprises at least one of an event where a preset time period arrives, an event where the evaluation score reaches a preset threshold point, an event where a user check signal for checking the evaluation score is input, and an event where a request for checking the evaluation score is input from an external device.

6.  The user customized health management method of any preceding claim, wherein the health care information comprises new behavior guideline information extracted based on the comparison of the behavior guideline and the behavior information.

7.  The user customized health management method of any preceding claim, wherein the health care information comprises a temporary behavior guideline extracted based on the comparison of the behavior guideline and the behavior information.

8.  The user customized health management method of claim 7, further comprising:

sending the temporary behavior guideline to an external device; and
replacing the behavior guideline with the temporary behavior guideline after receiving approval information of the temporary behavior guideline from the external device

9. The user customized health management method of any preceding claim, wherein the behavior guideline comprises at least one of a first behavior guideline effective with approval, and a second behavior guideline effective without approval.

10. The user customized health management method of any preceding claim, further comprising:

sending the health care information to an external device,
wherein the external device comprises at least one of a system server, a health management server, a hospital server, a insurance company server, a user terminal device, and a network server.

11. The user customized health management method of any preceding claim, wherein the user terminal device reads data relating to the behavior of the user from a short-range wireless communication tag when an external object attached with a short-range wireless communication tag is accessed by the user terminal device.

12. A user customized health management method for a system server, the method comprising the steps of:

receiving a behavior guideline for a user of a first device from a second device and forwarding the behavior guideline to the first device;
receiving behavior information from the first device;
generating health care information by comparing the behavior information and the behavior guideline;
sending the health care information to the second device and receiving a new behavior guideline from the second device; and
forwarding the new behavior guideline to the first device.

13. A health management system for user customized health management, the system comprising:

a first device;
a second device for providing a behavior guideline for a user of the first device; and
a system server for forwarding the behavior guideline to the first device when receiving the behavior guideline from the second device, and generating health care information by comparing the behavior information and the behavior guideline when receiving behavior information from the first device.
wherein the system server is configured for sending the health care information to the second device, receiving a new behavior guideline from the second device, and forwarding the new behavior guideline to the first device.

14. A user terminal device comprising:

a storage unit for storing a behavior guideline;
a communication reader (e.g. short-range wireless communication reader) for reading data recorded in a tag (e.g. short-range wireless communication tag) when at least one external object attached with a tag (e.g. short-range wireless communication tag) is accessed; and
a controller for generating behavior information according to the information read by the reader, generating health care information by comparing the behavior information and the behavior guideline, and storing the health care information in the storage unit.

15. A system server comprising:

a communication unit for receiving a behavior guideline of a user of a first device from a second device;
a controller for controlling the communication unit to forward the behavior guideline to the first device, and when receiving behavior information from the first device, generating health care information by comparing the behavior information and the behavior guideline; and
a storage unit for storing the health care information,
wherein the controller is configured for controlling the communication unit to send the health care information to the second device and, when receiving a new behavior guideline from the second device, to forward the new behavior guideline to the first device.

# FIG. 1

# FIG. 2

100

110

120

130

| SHORT-RANGE WIRELESS COMMUNICATION READER | ◄──► | CONTROLLER | ◄──► | STORAGE |

# FIG. 3

# FIG. 4

```
                                          S410              FIRST DEVICE    ~100
(FOOD CONTAINER)    SHORT-
                    RANGE
FIRST OBJECT ◄──    ACCESS                            ▼
                                          STORE FOOD INTAKE  ~S415
         10                               INFORMATION

                                                 ▼
              N              NORMAL FOOD
        ┌──────────────────◄ INTAKE?  ◄──── S420
        │    S430                 │ Y
        ▼                         ▼
   ADD EVALUATION          ADD EVALUATION   ~S425
   SCORE B                 SCORE A
        │                         │
        └────────────┬────────────┘
                     ▼
            GENERATE HEALTH   ~S435     S440    20
            CARE INFORMATION         SHORT-         SECOND
                     │               RANGE          OBJECT
                     ▼               ACCESS
            STORE MEDICATION  ~S445          (MEDICINE BOTTLE)
            INFORMATION

                     ▼
         N              NORMAL
   ┌────────────────◄  MEDICATION? ◄──── S450
   │   S460                  │ Y
   ▼                         ▼        S455
ADD EVALUATION          WITHIN          N
SCORE E              ADMINISTRATION ─────────┐
                        TIME?               │
                           │ Y              │
                           ▼     S465       ▼    S470
                    ADD EVALUATION    ADD EVALUATION
                    SCORE C           SCORE D
   └─────────────────────┬─────────────────┘
                         ▼
                UPDATE HEALTH    ~S475
                CARE INFORMATION
         S485            ▼
                     Y
   OUTPUT ◄───────    EVENT?   ◄──── S480
                        │ N
                        ▼
```

# FIG. 5

1000

| 1100 | 1200 | 1300 |
|------|------|------|
| COMMUNICATION UNIT | CONTROLLER | STORAGE |

# FIG. 6

# FIG. 7

100 FIRST DEVICE

1000 SYSTEM SERVER

200 SECOND DEVICE

INPUT BASIC INFORMATION — S710

SEND BASIC INFORMATION — S715

GENERATE BEHAVIOR GUIDELINE — S720

S725 — REQUIRE APPROVAL?

N

Y — SEND — S730

S735 — APPROVE

SEND — S740

SEND BEHAVIOR GUIDELINE — S745

GENERATE BEHAVIOR INFORMATION — S750

GENERATE HEALTH CARE INFORMATION — S755

S760 — EVENT?

N

Y — SEND HEALTH CARE INFORMATION — S765

SEND HEALTH CARE INFORMATION — S770

# FIG. 8

| FIRST DEVICE 100 | SYSTEM SERVER 1000 | SECOND DEVICE 200 |

INPUT BASIC INFORMATION — S810

SEND

S815

GENERATE BEHAVIOR GUIDELINE — S820

S825

N ◄ REQUIRE APPROVAL? ► Y    SEND    S830    S835

APPROVE

S840 SEND APPROVAL

SEND BEHAVIOR GUIDELINE

S845

GENERATE BEHAVIOR INFORMATION — S850

SEND BEHAVIOR INFORMATION

S855

GENERATE HEALTH CARE INFORMATION — S860

S865

N ◄ EVENT?

S875    SEND    Y    SEND    S870

# FIG. 9

100         1000         200

| FIRST DEVICE | SYSTEM SERVER | SECOND DEVICE |

INPUT BASIC INFORMATION ~S910

S915
SEND →

S920
SEND →

S925

GENERATE BEHAVIOR GUIDELINE

S930
S935 SEND ←
SEND ←

STORE BEHAVIOR GUIDELINE ~S940

GENERATE BEHAVIOR INFORMATION ~S945

GENERATE HEALTH CARE INFORMATION ~S950

SEND HEALTH CARE INFORMATION →
S955

STORE ~S960

S965
EVENT? N / Y

SEND HEALTH CARE INFORMATION →
S970

S975

CHANGE BEHAVIOR GUIDELINE

S980
S985 SEND ←
SEND ←

UPDATE BEHAVIOR GUIDELINE ~S990

# FIG. 10

# FIG. 11

100                          1000                          200

| FIRST DEVICE | | SYSTEM SERVER | | SECOND DEVICE |

INPUT BASIC
INFORMATION — S1110

SEND BASIC INFORMATION
→
S1115          SEND BASIC INFORMATION          S1125
→
S1120

GENERATE BEHAVIOR
GUIDELINE

S1130
SEND BEHAVIOR GUIDELINE

SEND BEHAVIOR GUIDELINE
←                                   ←

STORE BEHAVIOR — S1140          S1135
GUIDELINE

GENERATE BEHAVIOR
INFORMATION — S1145

SEND BEHAVIOR INFORMATION
→

S1150

GENERATE HEALTH
CARE INFORMATION — S1155

SEND HEALTH CARE INFORMATION          S1165
→

S1160

GENERATE NEW
BEHAVIOR GUIDELINE

SEND NEW BEHAVIOR GUIDELINE   SEND NEW BEHAVIOR GUIDELINE
←                                   ←

S1175          S1170

UPDATE BEHAVIOR — S1180
GUIDELINE

# FIG. 12

| 100 | 1000 | 200 | 300 | 400 |
|---|---|---|---|---|
| FIRST DEVICE | SYSTEM SERVER | SECOND DEVICE | THIRD DEVICE | FOURTH DEVICE |

S1210

INPUT BASIC
INFORMATION

SEND

S1215

SEND

S1225

S1220

GENERATE BEHAVIOR
GUIDELINE

S1230

S1235

SEND

SEND

S1240

SEND

S1245

GENERATE
INSURANCE DATA

STORE BEHAVIOR
GUIDELINE S1250

GENERATE BEHAVIOR
INFORMATION S1255

SEND

S1265

S1260

GENERATE HEALTH
CARE INFORMATION

S1270

SEND

S1275

SEND

S1280

GENERATE NEW
BEHAVIOR GUIDELINE

S1285

SEND

S1290

SEND

S1295

SEND

S1310

UPDATE BEHAVIOR
GUIDELINE S1300

S1305

UPDATE
INSURANCE DATA

N

EVENT?

SEND

Y

S1320

SEND

S1325

SEND

S1315

SEND

S1330

# FIG. 13

100

110

SHORT-RANGE
WIRELESS
COMMUNICATION UNIT

140

INPUT

120

CONTROLLER

150

OUTPUT

160

COMMUNICATION
UNIT

130

STORAGE

# FIG. 14

USER BASIC INFORMATION INPUT    ⬜ ⬜ ⊠

NAME [AAA]

RESIDENT REGISTRATION NUMBER [AAA-BBBB]

GENDER [male]    HEIGHT [180] cm

AGE [30]    WEIGHT [100] kg

GOAL WEIGHT [80] kg

EXERCISE PERIOD [30] 일

[MODIFY] [DONE]

~1500-1

1501    1502

CREATE BEHAVIOR GUIDELINE    ⊠

STATUS : EXTREMELY OBESE

BEHAVIOR GUIDELINE 1
MEAL : 600KCAL - 3 BOWLS OF RICE A DAY

BEHAVIOR GUIDELINE 2
SELECT EXERCISE TYPE

[WALKING ▼]  [WALK 4KM FOR ONE HOUR A DAY]

~1500-2

1503    1504    1505

# FIG. 15

200

210

**BEHAVIOR GUIDELINE INPUT**    ▭ ▭ ☒

PATIENT NAME    [AAA]    GENDER    [        ]    MANAGEMENT NUMBER    [        ]

BIRTH DATE    [        ]    BLOOD TYPE    [        ]    .....

}— 211

PATIENT STATUS

DISEASE    [        ]    STATUS    [        ]    CARE CYCLE    [        ]

.....

}— 212

PRESCRIPTION

MEDICATION    ROUTE OF MEDICATION    DOSAGE

[        ]    [            ]    [        ]    .....

[        ]    [            ]    [        ]    .....

}— 213

[ ADD ]    [ MODIFY ]    [ DONE ]

214

# FIG. 16

PATIENT STATUS CARE    310    200

311

| PATIENT NAME | AAA | GENDER | | MANAGEMENT NUMBER | |

BIRTH DATE          BLOOD TYPE          .....

312
PATIENT STATUS

HYPERTENSION

.....

BEHAVIOR GUIDELINE

A TAKE 10ML AFTER EVERY MEAL

B TAKE 1 PILL AFTER BREAKFAST

313

HEALTH CARE INFORMATION    EVALUATION SCORE

A   100% TAKE       100POINTS
B   100% TAKE       100POINTS

314

MODIFY BEHAVIOR GUIDELINE       DONE       PRINT

315-1          315-2      315-3

# FIG. 17

1600

| PATIENT STATUS CARE | ▭ ▢ ✕ |
|---|---|

**<BASIC INFORMATION>**

PATIENT NAME [AAA]   GENDER [male]   MANAGEMENT NUMBER [123456]

BIRTH DATE [1970.01.01]   BLOOD TYPE [A]   .....

1610

**<BEHAVIOR GUIDELINE1>**

MEDICATION        ROUTE                    DOSAGE

A   TAKE THREE TIMES IN TOTAL AFTER MEALS  10ml
B        TAKE 1 PILL AFTER BREAKFAST      1 PILL

1620

**<BEHAVIOR GUIDELINE2>**

EXERCISE    VOLUME      TIME

WALK          2km        1HOUR

1630

**<HEALTH CARE INFORMATION>**

ADMINISTRATION                    EXERCISE

A    100% ADMINISTERED  100POINTS    NO EXERCISE    0POINTS
B     50% ADMINISTERED   50POINTS

1640

| PRINT | SEND | MODIFY | DONE |
|---|---|---|---|

1651    1652    1653    1654

# FIG. 18

2012/4/01 SUNDAY
AM 9:00

YOUR HEALTH CARE
SCORE IS 90.

100

1800

# FIG. 19

# FIG. 20

2012/4/01 SUNDAY
AM 9:00
A THE MEDICINE BOTTLE IS TAGGED.
DID YOU TAKE THE MEDICATION?

100

2000

2010 — Yes    No — 2020

# FIG. 21

100

2012/4/01 SUNDAY
AM 9:00

2100

IT IS TIME TO TAKE THE
MEDICATION.
PLEASE TAKE 1 PILL OF A.

# FIG. 22

~2200

| MANAGE |
|---|
| SET |
| MODE CHANGE |

→

~2200-1

**BASIC INFORMATION INPUT**

NAME [　　　　]

→

~2200-2

**BASIC INFORMATION INPUT**

GENDER [MALE] [FEMALE]

AGE [　　] ▲▼

HEIGHT [　　] ▲▼

WEIGHT [　　] ▲▼

2201

~2200-4

2012/4/01 SUNDAY
AM 9:00

[EXERCISE TIME 10:00]

←

~2200

| MANAGE |
|---|
| SET |
| MODE CHANGE |

2203    2202

←

~2200-3

| SETUP DONE |
|---|
| MODIFY |

↓

~2200-5

2012/4/01 SUNDAY
AM 10:00

[TIME TO EXERCISE!]

→

**or**

↘

~2200-6

2012/4/01 SUNDAY
AM 11:00

CONGRATULATIONS
**YOU FINISHED THE EXERCISE.**
+10 POINTS

~2200-6

2012/4/01 SUNDAY
AM 11:00

LACK OF EXERCISE
-10 POINTS

# FIG. 23

# FIG. 24

# FIG. 25

TIME TO TAKE MEDICATION!
ASPIRIN 1 PILL.
EFFECT : PREVENT CARDIAC DISEASE

TAKE/OK      CANCEL

2510          2520

# FIG. 26

GOOD FOR YOU!
INCREASE THE EXERCISE POINT BY 1.

SEE HOW TO
FURTHER
INCREASE

OK

2610          2620

# FIG. 27

GOOD FOR YOU!

THE ACCUMULATED EXERCISE POINTS ARE OVER 15!
DO YOU LIKE TO RAISE THE INTENSITY?

| OK | CANCEL |
|----|--------|

2710        2720

# FIG. 28

OOPS!
EXERCISE POINT -1.

HOW TO INCREASE    OK

2810    2820

# FIG. 29

OOPS!
THE CUMULATIVE SCORES ARE BELOW 2.
PLEASE ASK A DOCTOR!

SEE HOW TO
INCREASE

OK

2910        2920

# FIG. 30

```
                    ┌─────────┐
                    │  START  │
                    └────┬────┘
                         ▼
        ┌────────────────────────────────────┐
        │    RECEIVE USER BEHAVIOR DATA       │──── S3010
        └────────────────┬───────────────────┘
                         ▼
        ┌────────────────────────────────────┐
        │   GENERATE BEHAVIOR INFORMATION     │──── S3020
        └────────────────┬───────────────────┘
                         ▼
        ┌────────────────────────────────────┐
        │ COMPARE THE PRESTORED BEHAVIOR      │──── S3030
        │ GUIDELINE AND BEHAVIOR INFORMATION  │
        └────────────────┬───────────────────┘
                         ▼
        ┌────────────────────────────────────┐
        │      GENERATE AND STORE HEALTH      │──── S3040
        │         CARE INFORMATION            │
        └────────────────┬───────────────────┘
                         ▼
                    ┌─────────┐
                    │   END   │
                    └─────────┘
```

# FIG. 31

START

USER INPUTS BASIC INFORMATION —S3110

GENERATE BEHAVIOR GUIDELINE —S3120

REQUIRE APPROVAL? —S3130
N
Y

SEND TO EXTERNAL DEVICE —S3140

APPROVED? S3150
N → DISCARD BEHAVIOR GUIDELINE S3160
Y

STORE BEHAVIOR GUIDELINE —S3170

END

# FIG. 32

START

RECEIVE BEHAVIOR GUIDELINE — S3210

SHORT-RANGE ACCESS? — S3220

N

Y

RECEIVE DATA — S3230

GENERATE BEHAVIOR INFORMATION — S3240

COMPARE BEHAVIOR GUIDELINE AND BEHAVIOR INFORMATION — S3250

GENERATE HEALTH CARE INFORMATION ACCORDING TO COMPARISON RESULT? — S3260

SEND HEALTH CARE INFORMATION — S3270

RECEIVE NEW BEHAVIOR GUIDELINE? — S3280

N

Y

UPDATE BEHAVIOR GUIDELINE — S3290

END

# FIG. 33

```
                    ( START )
                        |
                        v
    ┌──────────────────────────────────┐
    │ RECEIVE BEHAVIOR GUIDELINE FROM  │── S3310
    │          SECOND DEVICE           │
    └──────────────────────────────────┘
                        |
                        v
    ┌──────────────────────────────────┐
    │  FORWARD BEHAVIOR GUIDELINE TO   │── S3320
    │           FIRST DEVICE           │
    └──────────────────────────────────┘
                        |
                        v
    N         ◇ RECEIVE BEHAVIOR ◇ ─── S3330
   ┌──────────◇  INFORMATION?   ◇
   │              └────┬────┘
   │                   | Y
   │                   v
   │  ┌──────────────────────────────────┐
   │  │ COMPARE BEHAVIOR GUIDELINE AND   │── S3340
   │  │       BEHAVIOR INFORMATION       │
   │  └──────────────────────────────────┘
   │                   |
   └──────────────────>|
                       v
    ┌──────────────────────────────────┐
    │ GENERATE HEALTH CARE INFORMATION │── S3350
    └──────────────────────────────────┘
                       |
                       v
    ┌──────────────────────────────────┐
    │       SEND TO SECOND DEVICE      │── S3360
    └──────────────────────────────────┘
                       |
                       v
    N         ◇   RECEIVE NEW   ◇ ─── S3370
   ┌──────────◇ BEHAVIOR GUIDELINE? ◇
   │              └────┬────┘
   │                   | Y
   │                   v
   │  ┌──────────────────────────────────┐
   │  │       SEND TO FIRST DEVICE       │── S3380
   │  └──────────────────────────────────┘
   │                   |
   └──────────────────>|
                       v
                    (  END  )
```

# FIG. 34

# FIG. 35

# FIG. 36

```
        ┌──────────┐
        │  START   │
        └──────────┘
             │
             ▼
   ┌────────────────────┐
   │ GENERATE BEHAVIOR  │ ~S3610
   │    INFORMATION     │
   └────────────────────┘
             │
             ▼
   ┌────────────────────┐
   │ RECEIVE BIO-SIGNAL │ ~S3620
   └────────────────────┘
             │
             ▼
   ┌────────────────────┐
   │ GENERATE HEALTH CARE│ ~S3630
   │    INFORMATION     │
   └────────────────────┘
             │
             ▼
    N    ◇ ABNORMAL STATUS? ◇ ~S3640
             │ Y
             ▼
   ┌────────────────────┐
   │ OUTPUT ALARM SIGNAL│ ~S3650
   └────────────────────┘
             │
             ▼
        ┌──────────┐
        │   END    │
        └──────────┘
```

# FIG. 37

TAGGING

100

3000

# FIG. 38

3000

| 3010 | 3020 | 3030 | |
|------|------|------|---|
| SENSOR | EXERCISE VOLUME CALCULATOR | CONTROLLER | COMMUNICATION UNIT ~3050 |

3040

NFC MODULE

# FIG. 39

100

3910

SHORT-RANGE
WIRELESS
COMMUNICATION
READER

3950

STORAGE ⟷ CONTROLLER → MICROSERVER

3920          1000

SECOND
SENSOR ⟷ EXERCISE
VOLUME
CALCULATOR ~3930

3960

FIRST SENSOR —3940

# FIG. 40

# FIG. 41

100

| | |
|---|---|
| **160** BROADCASTING RECEIVER MODULE | POWER UNIT ~500 |
| **161** MOBILE COMMUNICATION MODULE | 120 |
| **162** | DISPLAY ~151 |
| **163** GPS MODULE | SPEAKER ~152 |
| **164** WIRELESS COMMUNICATION MODULE | STORAGE ~130 |
| **170** VOICE RECOGNITION PART | CONTROLLER |
| **180** MOTION RECOGNITION PART | EXTERNAL INPUT PORT 1 ~4200-1 |
| **4100** **4110** TOUCH SENSOR | EXTERNAL INPUT PORT 2 ~4200-2 |
| **4120** MAGNETOMETER | |
| **4130** ACCELEROMETER | EXTERNAL INPUT PORT N ~4200-n |
| **110** SHORT-RANGE WIRELESS COMMUNICATION UNIT | INPUT ~140 |

# FIG. 42

# FIG. 43

**WEB BROWSER MODULE** (4350)
- Web View
- Bookmark
- Download Agent
- Webkit

**Location**
- Location
- Landmark
- Map
- Route

**Social** (4360)
- Contacts
- Calendar
- Game

**SENSING MODULE** (4320)
- Sensor Data Analysis (4321)
  - color
  - location
  - User profile
- Face Recognition
- NFC Recognition
- Voice Recognition
- Gesture Recognition
- **DEVICE INFORMATION MANAGEMENT MODULE** (4322)
  - Device Info Provider
- **REMOTE CONTROL MODULE** (4323)
  - Capture
  - Phone
  - Print
  - TV

**Messaging** (4330, 4331)
- Instant Messenger
- SMS & MMS
- Email

**Telephony** (4332)
- Call Info Aggregator
- VoIP

**Multimedia** (4340, 4341)
- Player
- Camcorder
- Sound

**UI & Graphics** (4342, 4342-1)
- Image Compositor
- Coordinate Combination (4342-2)
- X11 (4342-3)
- 2D/3D UI Toolkit (4342-4)

**Storage** (4310, 4311)
- DB
- Registry

**Locale** (4312)
- Locale

**Security** (4313)
- Certificate
- Permission
- Secure Storage

**Network** (4314)
- DNET
- UPnP

EP 2 648 123 A1

**Europäisches Patentamt**
**European Patent Office**
**Office européen des brevets**

# EUROPEAN SEARCH REPORT

Application Number

EP 13 16 2518

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/161107 A1 (GOLDBERG NEAL [US] ET AL) 30 June 2011 (2011-06-30) * the whole document * ----- | 1-15 | INV. G06F19/00 |
| X | US 2007/033072 A1 (BILDIRICI NESIM N [US]) 8 February 2007 (2007-02-08) * the whole document * ----- | 1-15 | |
| X | US 2006/205564 A1 (PETERSON ERIC K [US]) 14 September 2006 (2006-09-14) * the whole document * ----- | 1-15 | |
| X | US 2002/120187 A1 (EIFFERT MICHAEL E [US] ET AL) 29 August 2002 (2002-08-29) * the whole document * ----- | 1-15 | |
| A | MARK HSIAO ET AL: "Intelligent Nutrition Service for Personalized Dietary Guidelines and Lifestyle Intervention", SERVICE SCIENCES (IJCSS), 2011 INTERNATIONAL JOINT CONFERENCE ON, IEEE, 25 May 2011 (2011-05-25), pages 11-16, XP031903137, DOI: 10.1109/IJCSS.2011.11 ISBN: 978-1-4577-0326-3 * the whole document * ----- | 11,14 | TECHNICAL FIELDS SEARCHED (IPC) G06F |

The present search report has been drawn up for all claims

| Place of search | Date of completion of the search | Examiner |
|---|---|---|
| Munich | 21 June 2013 | Sanandrés Ledesma, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 13 16 2518

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2013

| Patent document cited in search report | | Publication date | Patent family member(s) | | Publication date |
|---|---|---|---|---|---|
| US 2011161107 | A1 | 30-06-2011 | EP | 2008211 A2 | 31-12-2008 |
| | | | JP | 2009533729 A | 17-09-2009 |
| | | | US | 2011161107 A1 | 30-06-2011 |
| | | | WO | 2007117719 A2 | 18-10-2007 |
| US 2007033072 | A1 | 08-02-2007 | NONE | | |
| US 2006205564 | A1 | 14-09-2006 | US | 2006205564 A1 | 14-09-2006 |
| | | | WO | 2006094288 A2 | 08-09-2006 |
| US 2002120187 | A1 | 29-08-2002 | NONE | | |

EPO FORM P0459

For more details about this annex : see Official Journal of the European Patent Office, No. 12/82